# EUROPEAN PATENT SPECIFICATION

(11) **EP 0 478 715 B1**
(45) Date of publication and mention of the grant of the patent: **03.04.1996**
(21) Application number: 90917873.3
(22) Date of filing: 22.06.1990
(51) Int. Cl.: A61K 9/127

(54) **TARGETED LIPOSOMES AND METHODS FOR LIPOSOME-PROTEIN COUPLING**
ZIELLIPOSOMEN UND VERFAHREN ZUR KUPPLUNG VON LIPOSOMEN-PROTEINEN
LIPOSOMES CIBLES ET PROCEDES D'ACCOUPLEMENT DE LIPOSOMES-PROTEINES

(30) Priority: 23.06.1989 US 370650; 26.09.1989 US 412779
(43) Date of publication of application: 08.04.1992
(73) Proprietor: THE LIPOSOME COMPANY, INC., Princeton, NJ 08540 (US)
(72) Inventor: LOUGHREY, Helen, C., Dublin (IE); CULLIS, Pieter, R., Vancouver, British Columbia V6J 3R2 (CA); BALLY, Marcel, B., Bowen Island, British Columbia V0N I6O (CA); CHOI, Lewis, S., L., Burnaby, British Columbia V5A 2G1 (CA); WONG, Kim, F., Vancouver, British Columbia V6R 2V5 (CA)
(74) Representative: Warcoin, Jacques
(86) International application number: US9003582
(87) International publication number: WO9100289

(56) References cited:
- WO-A-86/00238
- WO-A-86/01102
- WO-A-86/04232
- US-A- 4 193 983
- US-A- 4 588 578
- US-A- 4 610 868
- US-A- 4 721 612
- US-A- 4 743 560
- US-A- 4 863 740
- US-A- 4 880 635
- US-A- 4 885 172
- THE JOURNAL OF BIOLOGICAL CHEMISTRY, vol. 257, no. 1, January 1982, pages 286- 288; F.J. MARTIN et al.: "Irreversible coupling of immunoglobulin fragments to preformed vesicles"
- G. GREGORIADIS: "Liposome Technology", vol. III, 1984, pages 127-135, CRC Press, Boca Raton, FL, US

## Description

The present invention relates to a method for synthesizing a reactive lipid including, for example, N-[4-(p-maleimidophenyl)-butyryl]phosphatidylethanolamine (MPB-PE) and related compositions. The compositions of the present invention are useful as coupling agents and may be incorporated into liposomes and subsequently coupled to proteins, cofactors and a number of other molecules.

The present invention further relates to lipids modified with SMPB and related cross-linking agents and the liposomes obtained by incorporated substantially pure reactive lipid, including MPB-PE, and related coupling compositions into lipids.

Protein-liposomes conjugates of the present invention may be used for therapeutic and diagnostic targeting of liposomes.

Protein-liposome conjugates of the present invention may have a trans-membrane potential across their membranes, and may be dehydrated. In addition, the conjugates may contain ionizable bioactive agents, for example, antineoplastic agents, and may be used in diagnostic assays.

The present invention relates to a general method for producing sized protein-liposome conjugates exhibiting enhanced blood circulation times. The present invention also relates to sized protein-liposome conjugate compositions produced by the method of the present invention. The conjugates of the present invention preferably range in size from about 30 nm to about 150 nm and exhibit favorable blood circulation times.

Protein-liposome conjugates of the present invention may be used for targeting the delivery of an active agent in vivo or in diagnostics.

Protein-liposome conjugates of the present invention may have a trans-membrane potential across their membranes, and may be dehydrated. In addition, the conjugates may contain ionizable bioactive agents, for example antineoplastic agents, and may be used in diagnostic assays.

### BACKGROUND OF THE INVENTION

Liposomes are completely closed structures comprising lipid bilayer membranes containing an encapsulated aqueous volume. Liposomes may contain many concentric lipid bilayers separated by an aqueous phase (multilamellar vesicles or MLVs), or alternatively, they may comprise a single membrane bilayer (unilamellar vesicles). The lipid bilayer is composed of two lipid monolayers having a hydrophobic "tail" region and a hydrophilic "head" region. In the membrane bilayer, the hydrophobic (non-polar) "tails" of the lipid monolayers orient toward the center of the bilayer, whereas the hydrophilic (polar) "heads" orient toward the aqueous phase. The basic structure of liposomes may be made by a variety of techniques known in the art.

Liposomes have typically been prepared using the process of Bangham et al., (1965 J. Mol. Biol., 13: 238-252), whereby lipids suspended in organic solvent are evaporated under reduced pressure to a dry film in a reaction vessel. An appropriate amount of aqueous phase is then added to the vessel and the mixture agitated. The mixture is then allowed to stand, essentially undisturbed for a time sufficient for the multilamellar vesicles to form. The aqueous phase entrapped within the liposomes may contain bioactive agents, for example drugs, hormones, proteins, dyes, vitamins, or imaging agents, among others.

Liposomes may be reproducibly prepared using a number of currently available techniques. The types of liposomes which may be produced using a number of these techniques include small unilamellar vesicles (SUVs) [See Papahadjopoulos and Miller, Biochem. Biophys. Acta., 135, p. 624-638 (1967)], reverse-phase evaporation vesicles (REV) [See U.S. Patent No. 4,235,871 issued November 25, 1980], stable plurilamellar vesicles (SPLV) [See U.S. Patent No. 4,522,803, issued June 11, 1985], and large unilamellar vesicles produced by an extrusion technique as described in copending U.S. Patent Application Serial No. 622,690, filed June 20, 1984, Cullis et.al., entitled "Extrusion Technique for Producing Unilamellar Vesicles".

Liposomes may be used as carriers for a wide variety of materials, for example drugs, cosmetics, diagnostic reagents and bioactive compounds, among others. Liposome compositions to which proteins are conjugated may be designed for both diagnostic and in vivo uses. For example, the ability to produce an antibody-directed vesicle would be a distinct advantage over similar undirected systems (Gregoriadis, G., Trends Pharmacol Sci, 4, p. 304-307, 1983), as would the targeting of a specific receptor or other cell surface feature. Useful applications of these protein-liposome conjugates would be in the selective targeting of cytotoxic compounds entrapped in vesicles to circulating tumor cells (Wolff et.al., Biochim. Biophys. Acta, 802, p. 259-273 1984), or applications of these immunoglobulin-associated vesicles in the development of diagnostic assays. Further applications could result from the targeting of a specific protein-receptor interaction for delivery of active agent to a specific site in a patient. Indeed, protein conjugated liposomes theoretically could be used to target the delivery of any active agent to a site in the patient's system to which the protein will bind. Numerous techniques for the conjugation of proteins to liposomes have already been developed for a variety of purposes including the targeting of drugs via immunoliposomes [See Leserman, et al., Nature, 288, 602 (1980), Heath, et al., Proc. Natl. Acad. Sci. USA, 80, 1377 (1983) and Huang, et al., J. Biol. Chem., 258, 14034 (1983)], diagnostic protocols [See Ishimori, et al., J. Immunol. Methods, 75, 351 (1984) and Rodney, et al., J. Immunol., 134, 4035 (1985)] and liposomal vaccines [See Allison, et al., Nature, 252, 252 (1974)].

Liposomes may be covalently coupled to proteins, antibodies and immunoglobulins. Heath et.al. (Biochim. Biophys. Acta., 640, p. 66-81, 1981), describe the covalent attachment of immunoglobulins to liposomes containing glycosphingolipid. Leserman et. al. (Liposome Technology, III, 1984, CRC Press, Inc., CA., p. 29-40; Nature, 288, p. 602-604, 1980) and Martin et. al., (J. Biol. Chem., 257, p. 286-288, 1982) have described procedures whereby thiolated IgG or protein A is covalently attached to lipid vesicles, and thiolated antibodies and Fab' fragments are attached to liposomes, respectively. These protocols and various modifications (Martin et.al, Biochemistry, 20, p. 4229-4238, 1981; and Goundalkar et.al., J. Pharm. Pharmacol., 36, p. 465- 466, 1984) represent the most versatile approaches to coupling. Avidin-coupled and avidin and biotinyl-coupled phospholipid liposomes containing actinomycin D have successfully targeted tumor cells expressing ganglio-N-triosylceramide (Urdal et.al., J. Biol. Chem., 255, p. 10509-10516, 1980). Huang et.al. (Biochim. Biophys. Acta., 716, p. 140-150, 1982) demonstrate the binding of mouse monoclonal antibody to the major histocompatibility antigen H-2 (K), or goat antibody to the major glycoprotein of Molony Leukemia Virus, to palmitic acid. These fatty acid modified IgGs were incorporated into liposomes, and the binding of these liposomes to cells expressing the proper antigens characterized. Other in vitro efforts to specific binding of liposomes coated with specific immunoglobulins have been performed (Sharkey et.al., Fed. Proc., 38, 1089, 1979). In still other coupling studies, Rahman et. al. found that tissue uptake of liposomes could be altered by attachment of glycolipids to the liposomes (J. Cell Biol., 83, p. 268a, 1979).

In accordance with a primary use for liposomes, the entrapment of antineoplastic agents inside liposomal bilayers has resulted in more efficacious therapy as compared to direct administration of the drug. (Forssen et.al., Cancer Res., 43, p. 546, 1983; and Gabizon et.al., Cancer Res., 42, p. 4734, 1982). A major problem with the encapsulation of antineoplastic drugs as well as other agents is that many of these drugs have been found to be rapidly released from liposomes after encapsulation. This is an undesirable effect, in view of the fact that toxicity of many of the antineoplastic agents can be significantly reduced through liposome encapsulation as compared to direct administra- tion. See, for example, Forssen et.al. Cancer Res. 43, 546 (1983) and Rahman et.al. Cancer Res., 42, 1817 (1982). In addi- tion, certain pharmacological agents which are favorably delivered in sustained released fashion are not accommodated by standard liposomal delivery systems; many liposomal compositions release the agent too rapidly to provide sustained release delivery.

One answer to the above-described problem is the use of preformed, stable liposomes which maintain the stability and sustained release characteristics of the liposomal system. Liposomal compositions comprising protein-coupled liposomes have produced storage stable liposomes which may be stored stably for an indefinite period, as described in United States Patent Application, Serial Number 811,037, filed December 18, 1985, entitled "Novel Composition for Targeting, Storing and Loading of Liposomes". These liposomes, which include streptavidin and immunoglobulin coupled to liposomes, may be stored in a dehydrated state, with loading of the liposomes on an "as needed" basis. These protein-coupled liposomes have been loaded with ionizable antineoplastic agents wherein a trans-membrane potential is created across the walls of the liposomes and the antineoplastic agent is loaded into the liposomes by means of the trans-membrane potential. See, for example, U.S. Patent Application Serial No. 749,161, Bally et.al. entitled "Encapsulation of Antineoplastic Agents in Liposomes," filed June 26, 1985

As explained above, protein-liposome conjugates have many potential applications, ranging from diagnostic systems to the targeting of disease states in vivo. As indicated elsewhere [Loughery,et al., Biochim. Biophys. Acta., 901, 157 (1987], the coupling of streptavidin to liposomes results in a flexible basic system which subsequently allows the straightforward conjugation of a wide variety of proteins. However, liposome-protein conjugates tend to aggregate during the conjugation process, particularly at high protein to lipid ratios. For example, it has been found that increased amounts of protein [F(ab) fragments] conjugated to liposomes resulted in an increase in the polydispersity of vesicle populations [See Bredehorst R., et al., Biochemistry, 25, 5693 (1986)]. It has also been observed that conditions which increase the coupling efficiency of protein to liposomes, such as increasing the lipid concentration and the ratio of protein to lipid in the coupling incubation step, increase the extent of vesicle-aggregation as observed by negative staining [See Heath, et al., Biochim. Biophys. Acta, 599, 42 (1980)].

Aggregation of protein-liposome conjugates during protein coupling, unfortunately, is a characteristic which impairs the general applicability of this system. This aggregation phenomenon is associated with an increased size of liposomes. It has been observed that the rate of clearance of liposomes from the circulation is dependent on the size of the preparation; the larger the liposome, the faster it is removed from the circulation [See Hunt, A.C., Biochim. Biophys. Acta, 719, 450 (1982) and Sota, et al., Chem. Pharm. Bull., 34, 4244 (1986)]. Because of the tendency of protein liposome conjugates to aggregate, the size of such preparations has tended to be large and thus, circulation times have been somewhat disadvantageous. The clearance of protein-liposome conjugates from the blood has tended to be greater than non-conjugated liposomes of the same size. In addition, aggregated protein-liposome conjugates tend to be poorly taken up by cells via an endocytosis process which may diminish the amount of agent which enters the cells. In diagnostics, the aggregated conjugates tend to precipitate out of solution resulting in potential inaccuracies in diagnosis.

There is, therefore, a need in the art for a general method for producing protein-liposome conjugates of defined size distribution which may be utilized for general targeting applications. Such sized protein-liposome conjugates would be expected to show the favorable characteristics of protein-liposome formulations for targeting active agent delivery, including high cell uptake, or for use in diagnostics, without exhibiting substantial precipitation of aggregated protein-liposome conjugates.

It is an object of the present invention to provide a general method of attaching protein molecules to liposomes to achieve well-characterized sized protein-liposome conjugate systems for general targeting applications.

It is an additional object of the present invention to present a technique for the generation of sized protein-liposome conjugates which should allow ease of conjugating protein to liposome without affecting the binding activity of the protein to which the liposome is conjugated.

It is a further object of the present invention to provide a general method for the generation of protein-liposome conjugates of defined size distribution which can accommodate varying amounts of protein.

It is still a further object of the present invention to provide stable protein-liposome conjugates which are produced by the method of the present invention.

It is still an additional object of the present invention to provide a general method to allow for easy manipulation of the physical size of protein-coupled liposomes without affecting the binding activity of the protein.

It is yet another object of the present invention to enhance the efficiency of the production of sized protein liposome conjugates by providing an efficient coupling technique in combination with stable cross-linkages to increase the in vitro capabilities and stability of the conjugates to more efficiently deliver encapsulated materials to cells.

It is a further object of the present invention to provide sized protein-liposome conjugates which can be stored stably for long periods of time.

It is still another object of the present invention to provide sized protein-liposome conjugates which may be loaded with a bioactive agent using a trans-membrane ion potential.

Covalent attachment of liposomes to antibodies which are directed against cell surface antigens such as those associated with transformed cells, has considerable therapeutic potential. However, at the present time, such targeted liposomal systems have mainly been used for in vitro applications such as in diagnostic assays (Martin and Kung, Ann. N.Y. Acad. Sci., pp. 443-449 (1985). In order to exploit the full potential of antibody targeted carrier systems, as well as other systems, for example, liposome-protein coupling and liposome-cofactor coupling, an improved versatile and reliable methodology for coupling should be developed.

To date, no general procedure for attaching proteins, antibodies and other molecules to liposomes is yet available. Leserman, et al. Nature (London), 288, 602-604 (1980) and Barbet, et al., J. Supramol. Struct. Cell. Biochem., 16, 243-258 (1981) have described a procedure wherein a thiolated IgG is covalently attached to liposomes containing N-[3-(2-pyridyldithio)-propionyl] phosphatidylethanolamine (PDP-PE) via a disulfide bond. A more general version of this procedure was developed by coupling protein A to vesicles (see, for example, Leserman (1980), supra.), which takes advantage of the ability of protein A to bind the Fc portion of IgGs of certain classes. One major limitation of this method is that many monoclonal antibodies are not of the appropriate class.

An alternative to the above approach to coupling is that of Martin and Papahadjopoulos, J. Biol. Chem., 257, 286-288 (1982) who developed the technique of covalently attaching antibodies and Fab' fragments to liposomes containing N-[4-(p-maleimidophenyl)-butyryl] phosphatidylethanolamine (MPB-PE) by formation of a thio-ether linkage with the maleimido group, a linkage which is considerably less susceptible to reducing conditions found in the serum than is the disulfide linkage of the Leserman method. The Martin/Papahadjopoulos approach as well as various modifications of this approach [see, for example, Wolff and Gregoriadis, Biochem. Biophys Acta, 802, 259 (1984), Martin, et al., Biochemistry, 20, 4229 (1981) and Goundalkar, et al., J. Pharm. Pharmacol., 36, 465 (1984) represent the most versatile approaches to coupling currently available.

In this method of cross-linking liposomes to proteins, antibodies, cofactors and other molecules to liposomes, cross-linking agents containing a maleimido group, for example, N-succinimidyl 4-(p-maleimidophenyl)butyrate (SMPB), among others, are used to cross-link phosphatidylethanolamine and other amine containing lipids to thiol containing conjugated molecules, for example, proteins, antibodies, cofactors and other molecules containing reactive thiols. Prior art cross-linking agents, for example SMPB, which are reacted with phosphatidylethanolamine and other lipids according to literature protocols are subject to an opening of the maleimide ring during displacement of the succinimidyl group, resulting in contamination of the reacted product with the ring-opened MPB-lipid derivative. Cross-linking to proteins, antibodies, cofactors and other molecules is less than ideal using the prior art literature protocols. The method of the present invention serves to obviate this problem by providing liposomes comprising MPB-PE, i.e., SMPB derivatized phosphatidylethanolamine exhibiting an absence of ring-opened MPB-lipid which is produced using the prior art methods. Liposomes comprising pure MPB-PE may be further reacted with various proteins, for example, streptavidin, among others, antibodies, cofactors and other molecules to produce conjugated liposomes of the present invention.

In accordance with a primary aspect of the present invention, i.e., the delivery of bioactive agents to a therapeutic site, the entrapment of antineoplastic agents inside liposomal bilayers has resulted in more efficacious therapy as compared to direct administration of the drug. (Forssen et al., Cancer Res., 43, p. 546, 1983; and Gabizon et al., Cancer Res., 42, p. 4734, 1982). A major problem with the encapsulation of antineoplastic drugs as well as other agents is that many of these drugs have been found to be rapidly released from liposomes after encapsulation. This is an undesirable effect, in view of the fact that toxicity of many of the antineoplastic agents can be significantly reduced through liposome encapsulation as compared to direct administration. See, for example, Forssen et al., Cancer Res., 43, 546 (1983) and Rahman et al., Cancer Res., 42, 1817 (1982). In addition, certain pharmacological agents which are favorably delivered in sustained released fashion are not accommodated by standard liposomal delivery systems; many liposomal compositions release the agent too rapidly to provide sustained release delivery.

In accordance with the present invention, a conjugated liposome made by binding a protein, antibody, cofactor or other molecule ;to a liposome comprised of an effective amount of substantially pure MPB-PE and related maleimide containing derivatives may be stored stably for an indefinite period, in a dehydrated state, with loading of the liposomes on an "as needed" basis.

It is an object of the present invention to provide a general method for the synthesis of MPB-lipid and in particular, MPB-PE and related maleimide containing derivatives and related compounds.

It is an additional object of the present invention to provide liposomes comprising MPB-PE and related maleimide containing derivatives. Such liposomes may be further reacted with proteins, antibodies, cofactors and other molecules ;to produce conjugated liposomes.

It is still a further object of the present invention to provide conjugated liposomes of the present invention which have entrapped at least one bioactive agent, such as a drug.

It is still another object of the present invention to provide an efficient coupling technique in combination with stable cross-linkages to produce liposome conjugates which may more efficiently deliver encapsulated materials to cells.

It is yet an additional object of the present invention to provide stable conjugated liposomes which have more efficiently bound protein to the liposome than prior art methods and which can be stored stably for long period of time.

US Patent n° 4,743,560 is directed to the preparation of maleimide moiety-containing lipids by a method using a methanol-containing solvent resulting in ring-opened impure reactive lipid.

### SUMMARY OF THE INVENTION

In the method of the present invention, phosphatidylethanolamine (PE) or a related liposome forming nucleophilic lipid is reacted with a cross-linking agent having at least one maleimido group and an amine reactive function, for example, SMPB, to produce a reactive lipid, for example, MPB-PE. In the method of the present invention, the reaction of the nucleophilic lipid, for example, PE, occurs in the absence of hydrolytic conditions to avoid a ring-opened side product which is produced by following the method of the prior art. After the production of pure reactive lipid, for example, MPB-PE, a thiol-containing conjugated molecule, for example, a protein or other molecule such as an antibody or cofactor, is covalently linked to the reactive lipid to produce a liposome cross-linked with the protein, antibody, cofactor or other molecule. As used herein, such a cross-linked liposome is referred to as a conjugated liposome.

It has surprisingly been discovered that the reaction of SMPB with a nucleophilic liposome forming lipid, for example, PE, utilizing the prior art methodology (conditions which employ methanol, ethanol or other alcohols solvent under basic conditions) results in the production of a reactive lipid, for example, MPB-PE, plus a substantial amount of a side product in which the maleimide ring is opened by the alcohol ("ring-opened side product"). It has also been discovered that the use of chromatographic and other separation techniques employing alcohols results in the production of a significant amount of ring-opened side product.

In the method of the present invention to produce a reactive lipid, for example, MPB-PE of the present invention, the following steps are utilized:
1. A cross-linking agent such as SMPB is reacted with a nucleophilic lipid in a solvent containing a non-nucleophilic amine in the absence of a nucleophilic solvent for a period of time sufficient to complete conversion of nucleophilic lipid to reactive lipid such as MPB-PE;
2. After the reaction to form reactive lipid is substantially complete, the solution is diluted with a solvent and washed at least once with water, preferably a saline solution, to remove by-products; and
3. The solution from (2) is concentrated in vacuo and the solid residue triturated to remove unreacted SMPB and succinimide by-product to produce a reactive lipid, for example, MPB-lipid.

It is to be noted that substantially pure reactive lipid may also be made by employing steps 1 and 3 sequentially without step 2. Although many of the reactive lipids of the present invention are made using all three steps set forth hereinabove, it is understood that certain reactive lipids may be triturated from solution after conversion step 1 to produce substantially pure reactive lipid.

In further steps of the method aspect of the present invention, pure reactive lipid may be recrystallized and subsequently incorporated into liposomes to form reactive liposomes. The reactive liposomes may be reacted with a conjugated molecule, e.g., a thiol-containing protein, antibody, cofactor or a related molecule to produce a conjugated liposome of the present invention. The conjugated liposomes of the present invention. The conjugated liposomes of the present invention may be used for targeting the delivery of a wide variety of bioactive agents or for diagnostic purposes. For example, the application of these conjugated in targeting and diagnostic regimes may be illustrated by the specific binding of such conjugates to lymphocytes via defined biotinated monoclonal antibodies, in a manner which is reflective of the cell distribution of the target antigen [see, for example, Stashenko, et al., J. Immunol., 125, 1678 (1980) and Howard, et al., J. Immunol., 126, 2117 (1981)].

A new protocol for the synthesis of a pure SMPB derivative of a nucleophilic lipid is presented here. Coupling conditions for the conjugation of proteins to liposomes were optimized such that the integrity of the maleimide function of the reactive lipid, for example, MPB-Dipalmitoylphosphatidylethanolamine (MPB-DPPE), was retained. Coupling efficiencies of over 50% are readily achieved under the optimized conditions detailed in the present application. Similar efficiencies have been attained using the prior art methods only in conjunction with higher levels of MPB EPE in liposomes [5 mole %; see, for example, Bragman, et al., J. Natl. Cancer Inst., 73, 127 (1984)]. The efficient coupling associated with the use of reactive lipid, for example, MPB PE of the present invention, is of particular importance as concentrations of reactive lipid in liposomes of greater than about 2.5 mole % dramatically affect liposome stability [see, for example, Bradehorst, et al., Biochemistry, 25, 5693 (1986)].

The present invention is also directed to reactive liposomes comprising at least one reactive lipid, for example, MBP-PE, in combination with at least one additional liposome forming lipid. Liposomes of the present invention generally comprise at least about 0.05 mole percent of a reactive lipid such as MPB-PE and no greater than about 99.95 mole percent of at least one liposome producing lipid. Conjugated liposomes of the present invention further comprise various proteins, antibodies, cofactors and other molecules which are covalently or non-covalently linked to the liposomes. In one aspect of the present invention, streptavidin is used to form conjugated liposomes of the present invention. These streptavidin coated liposomes rapidly and efficiently bind biotinated proteins and lead to conjugated liposomes which exhibit specific targeting properties in vitro. Such liposomes may be utilized in therapeutic and diagnostic targeting applications.

In another method of the present invention, liposomes are linked to a protein, for example streptavidin or an immunoglobulin, among other proteins, via a covalent or non-covalent linkage to produce an aggregated protein-liposome conjugate. This aggregated, conjugated liposomal preparation is then extruded through a filter having a pore size ranging from about 30 nm to about 100 nm to produce sized protein-liposome conjugates. It has been found that the extrusion of the protein-liposome conjugate after coupling reverses the aggregation that is produced when proteins are coupled to liposomes to produce stable, non-aggregated protein-liposome conjugates of consistent size which do not readily reaggregate. It is a surprising result that the extrusion process occurs without filtering out the aggregated protein-liposome conjugates.

The method of the present invention allows easy manipulation of the physical size of protein-liposome conjugates and may avoid affecting the binding activity of the protein. Stable protein-liposome conjugates of defined size distribution can readily be prepared with various amounts of protein attached to the liposomes by this technique. The enhanced blood circulation times of extruded conjugates and the retention of binding capacity in the experiments performed indicate that extruded preparations of protein-coupled liposomes should be capable of binding to protein binding sites in vivo.

The present invention also relates to protein-liposome conjugates. Although the weight percentages of the various components of this aspect of the present invention may vary greatly, in general, the protein-liposome conjugates of the present invention comprise:
1. a liposome vesicle comprising:
   a). at least about 90 mole percent of a liposome producing lipid; and
   b). at least about 0.1 mole percent of a functionalized lipid; and
   c). a protein linked to said functionalized lipid in an amount equal to about 10 to about 100 protein molecules per liposome vesicle.

Liposomes of the present invention may be loaded with a bioactive agent as well as pharmaceutical agents, for example local anaesthetics, bronchodilators, beta-adrenergic blockers, antihypertensive agents, anti-depressants, anti-convulsants, anti-histamines, anti-malarial agents and analgesics among a number of other pharmaceutical agents. To load an active agent into the liposomes of the present invention, the liposomes are preferably prepared in such a way as to create a trans-membrane potential across their lamellae in response to a concentration gradient. This concentration gradient may be created by either a Na+/K+ potential or a pH gradient (H+). The difference in internal versus external potential is the mechanism which drives the loading of the liposomes with ionizable bioactive agents; delayed loading of preformed liposomes will occur in response to the trans-membrane potential.

The protein-liposome conjugates of the present invention may be dehydrated in the presence of one or more protecting sugars, stored in their dehydrated condition, and subsequently rehydrated with retention of the ion gradient and associated ability to accumulate the bioactive agent. In addition, the protein- liposome conjugates of the present invention may be used in diagnostic assays.

The liposome conjugates of the present invention may be dehydrated in the presence of one or more protecting sugars, stored in their dehydrated condition, and subsequently rehydrated with retention of the ion gradient and associated ability to accumulate the bioactive agent. In addition, the protein-liposome conjugates of the present invention may be used in diagnostic assays.

### BRIEF DESCRIPTION OF THE DRAWINGS

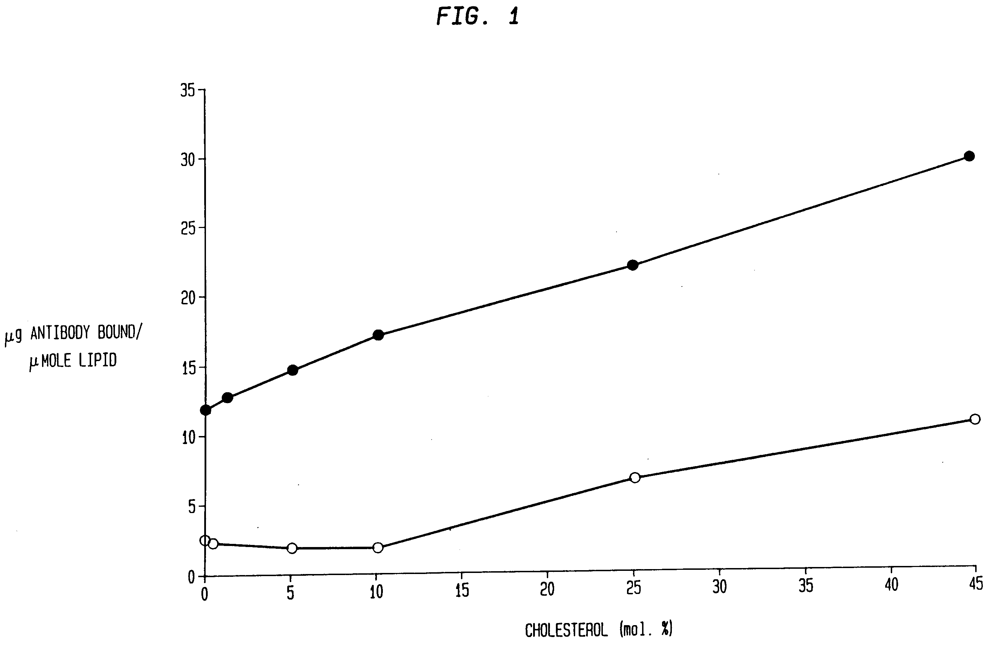

Figure 1 shows the comparison of coupling of thiolated IgG to liposomes containing PDP-EPE and MPB-EPE as a function of the concentration of cholesterol included in the liposomes As indicated, significant coupling of thiolated IgG to liposomes containing the PDP-EPE did not occur until greater than 20 mole % of cholesterol was incorporated into the liposomes. In start contrast, levels of 12 ug IgG/umole of lipid were obtained for liposomes containing MPB PE, even in the absence of cholesterol.

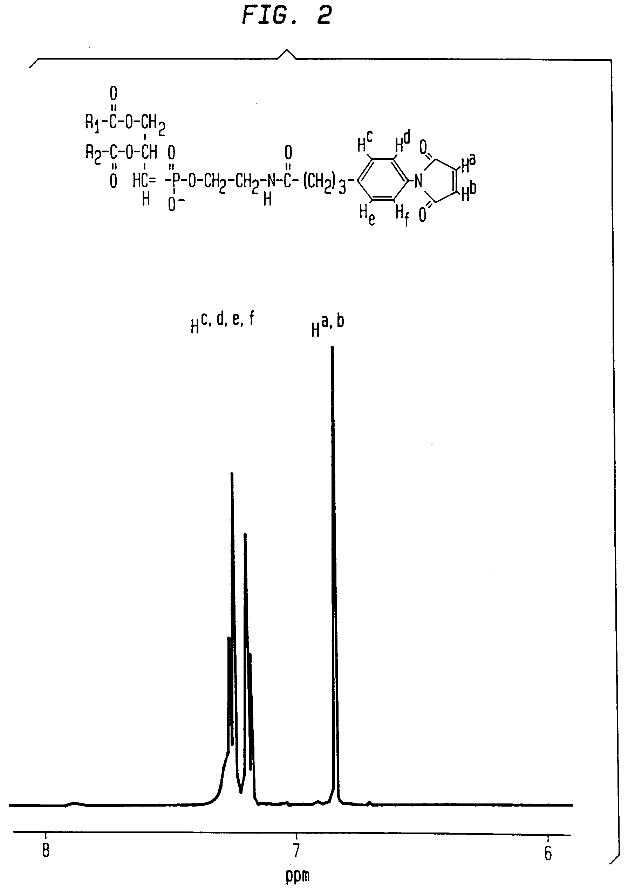

Figure 2 shows the NMR spectra of pure MPB-DPPE, which is synthesized by the method of the present invention.

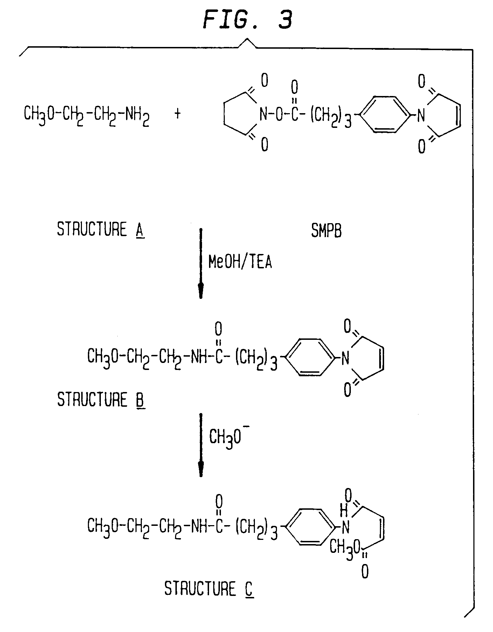

Figure 3 presents a structural representation of the proposed reaction scheme of 2-methoxyethylamine (for purposes of determining the structure of ring-opened side product, the chemical equivalent of PE) with SMPB under conditions to those described in the prior art for synthesis of MPB-PE. The figure indicates that the reaction produces two products, one similar in structure to MPB-PE (Structure B) and the other product similar in structure to the ring-opened side product produced by methanolic attack on the keto group of the maleimide group.

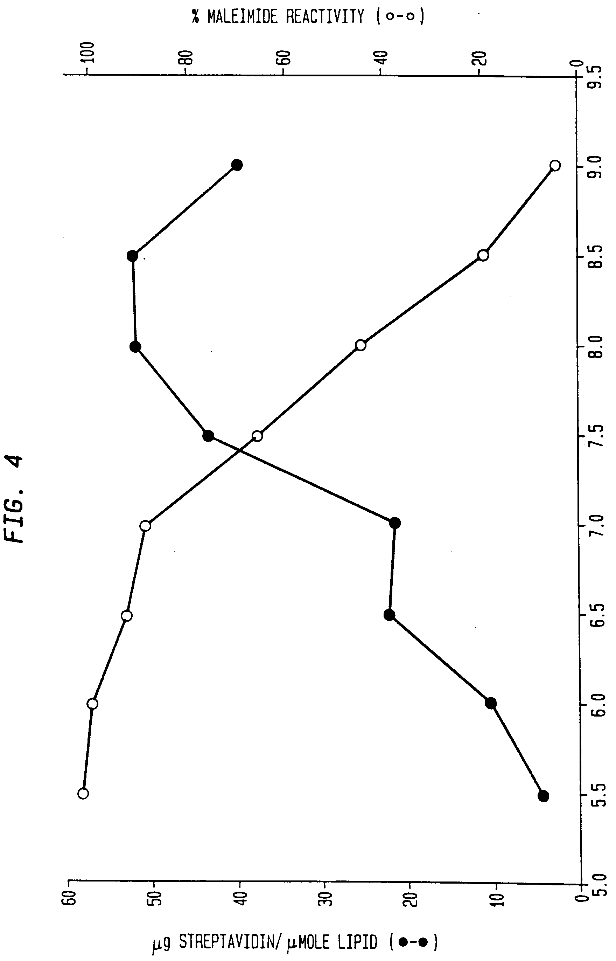

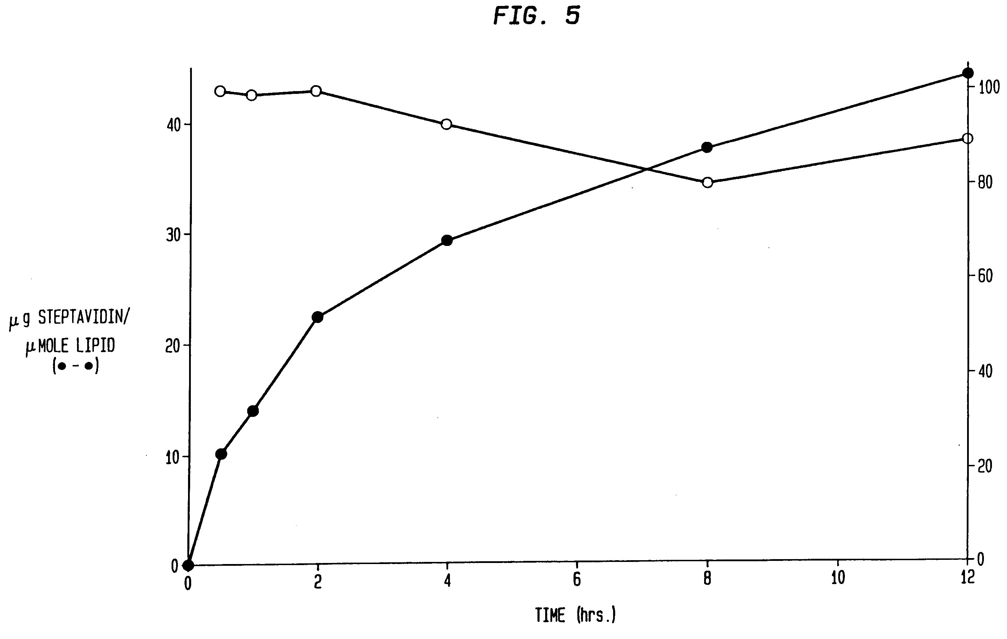

Figures 4 and 5 represent the investigation of the optimal conditions for coupling thiolated streptavidin to liposomes containing pure MPB DPPE. As shown in Figure 4, the amount of liposomally conjugated protein increased rapidly at pH values greater than 7.0, but resulted in a corresponding rapid degradation of the maleimide group of the reactive lipid. Figure 5 shows a time course reacting streptavidin binding to liposomes to the reactivity of the maleimide lipid. The results indicate that optimal levels of streptavidin conjugated to liposomes (approx. 37 ug/umole of lipid) were obtained with minimal degradation of the maleimide after an incubation period of 8 hours at pH 7.5 and room temperature.

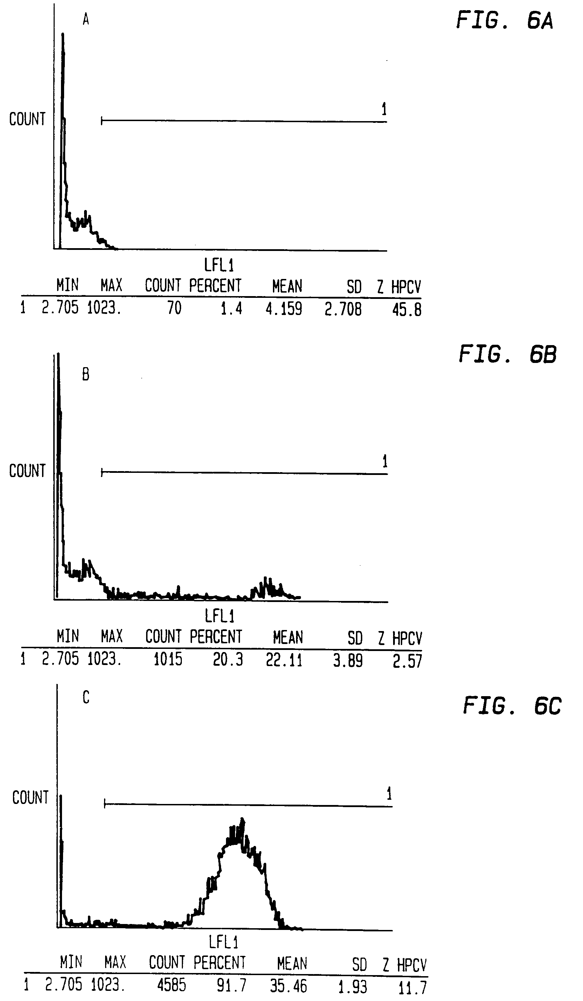

Figures 6A, B and C compare the targeting of liposomes to target cells through incubation of liposome streptavidin conjugates in the absence (Figure 6A) or the presence of biotinated antibodies (Figures 6B and C) measured by flow cytometry techniques.

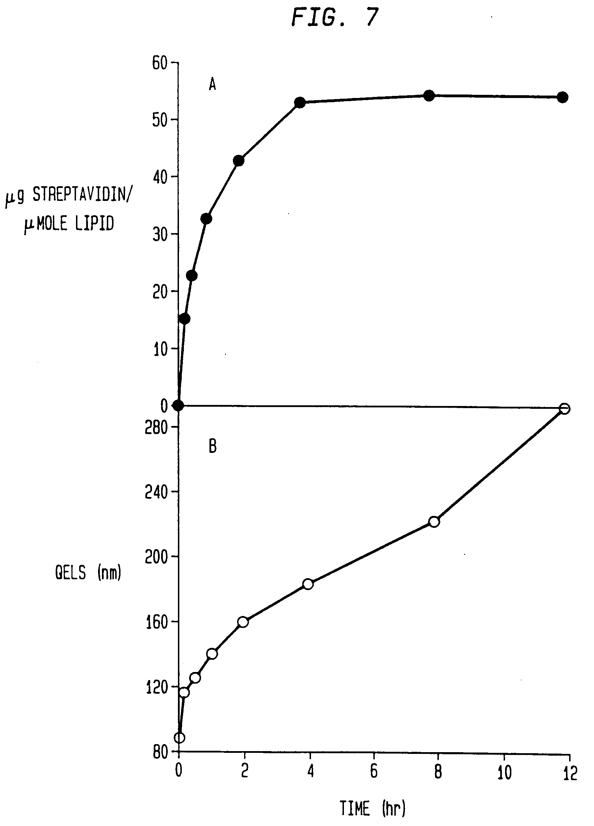

Figure 7 is a graph showing the effect on vesicle size of coupling streptavidin to liposomes. As described in Example 20, liposomes (54%EPC, 45% CHOL, 1% MPB-PE, 5 mM final lipid concentration, 100 nm) were incubated with streptavidin (100 ug protein/umole lipid) over time at pH 7.5. At various times as indicated on the graph, the reaction was quenched by the addition of N-ethylmaleimide (500 molar ratio to protein) and free streptavidin was removed by gel filtration on sepharose CL-4B. The extent of coupled streptavidin was determined by 3H biotin binding (graph A) and vesicle size gas estimated by QELS (Graph B).

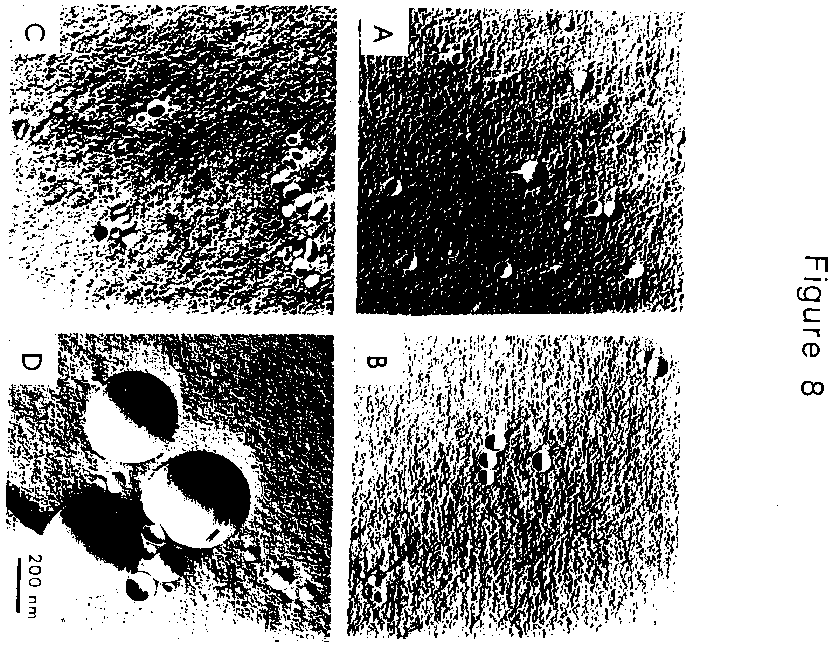

Figure 8 is a freeze fracture of streptavidin-liposome preparations. Streptavidin-liposome samples were quenched with N-ethylmaleimide at 0.2 (A), 2 (B), 4 (C) and 18 (D) hours prepared as described for Example 20 and figure 7, above and examined by freeze fracture.

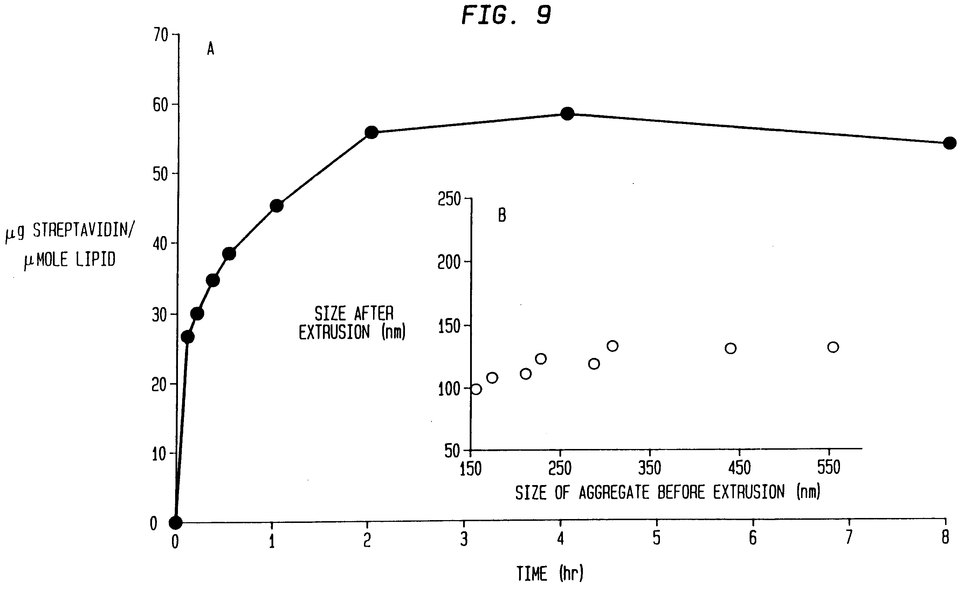

Figure 9 is a graph representing the extrusion of streptavidin- liposome conjugates as described in Example 21. Streptavidin was coupled to liposomes (100 nm) containing 1% MPB-PE at a final lipid concentration of 20 mM. At various times, aliquots of the reaction mixtures were quenched with N-ethylmaleimide and diluted to 5 mM lipid concentration before extrusion through 100 nm polycarbonate filters. The extent of coupled streptavidin (A) was estimated by 3H biotin binding to streptavidin liposomes after gel exclusion of lipid samples on sepharose CL-4B. The size of streptavidin-liposome conjugates was estimated by QELS before and after extrusion (B).

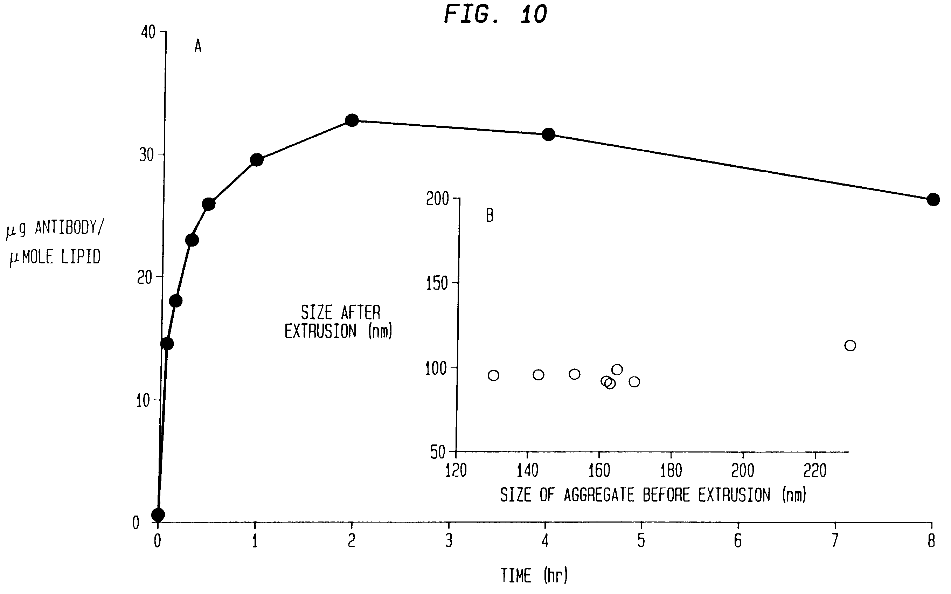

Figure 10 is a graph representing the extrusion of antibody liposome conjugates as described in Example 21. Fluorescein labeled antibody was coupled to liposomes (100 nm) containing 1% MPB-PE at a final lipid concentration of 20 mM. At various times aliquots of the reaction mixtures were quenched with N-ethylmaleimide and diluted to 5 mM lipid concentration before extrusion through 100 nm polycarbonate filters. The extent of coupled antibody (A) was determined by estimating the levels of liposomally associated fluorescence after exchange of lipid samples on sepharose CL-4B. The size of the antibody liposome was estimated by QELS before and after extrusion (b).

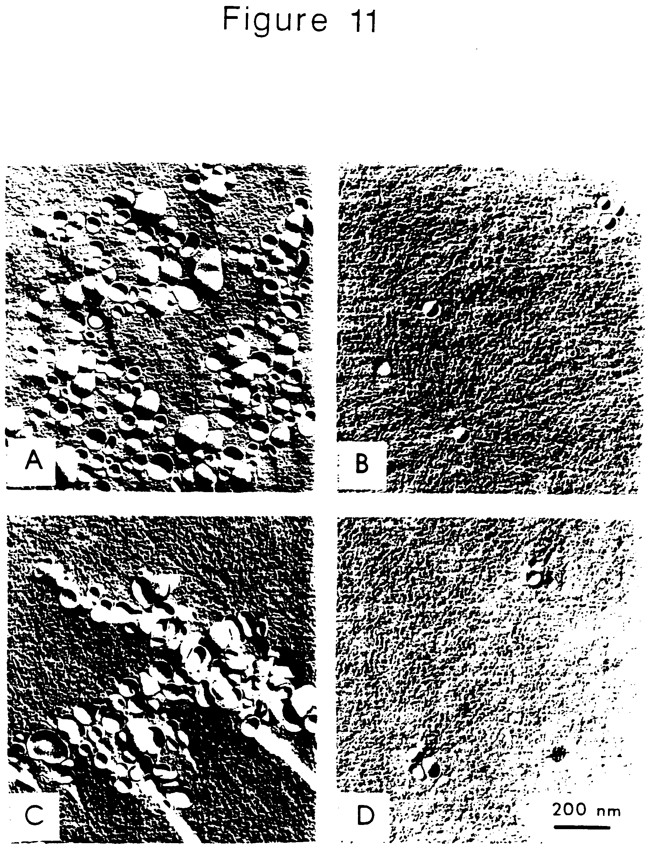

Figure 11 is a freeze fracture of streptavidin liposomes before and after extrusion as described in Example 21. Streptavidin was coupled to liposomes at a final lipid concentration of 20 mM for 8 hours as described in Figure 7 and Example 21. The sample was diluted to 5 umoles/ml prior to extrusion. Non-covalent attachment of streptavidin to liposomes containing biotin-PE (0.25%) was performed as described in Example 13 at a final lipid concentration of 5 mM. Samples were examined by freeze fracture before and after extrusion through 100 nm polycarbonate filters. Indicated in the figure are Streptavidin-liposomes containing MPB-PE before (A) and after (B) extrusion and Streptavidin-liposomes containing biotin-PE before (C) and after (D) extrusion.

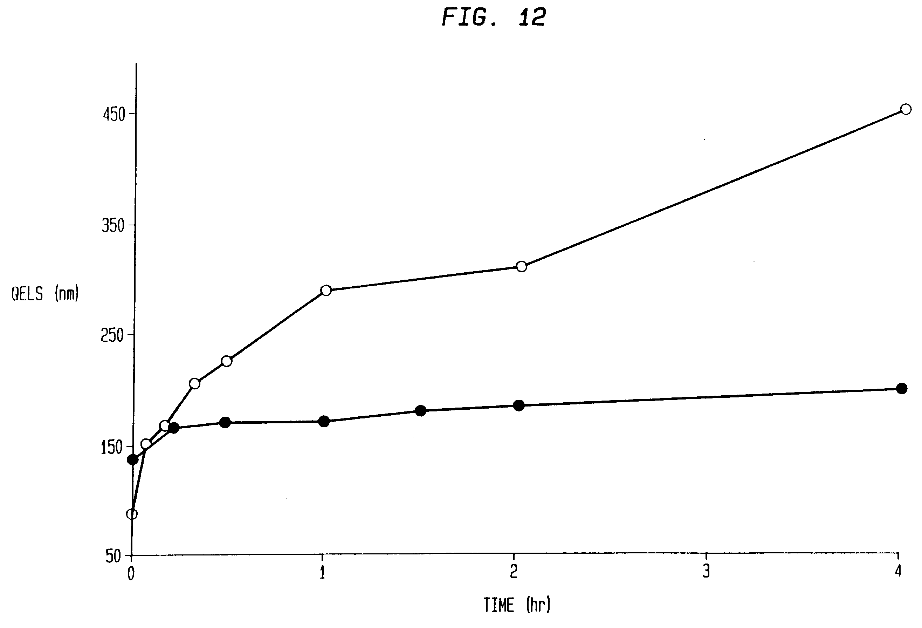

Figure 12 represents the examination of the stability of extruded streptavidin-liposome conjugates by QELS. Streptavidin-liposomes with approximately 51 ug protein/umole lipid were prepared by incubating thiolated streptavidin with liposomes containing 1% MPB-PE for 8 hours at a final lipid concentration of 20 mM. After removal of unbound streptavidin by gel filtration on sepharose CL-4B, the sample was diluted to 5 mM lipid and extruded 10 times through two stacked 100 nm polycarbonate filters. At various times as indicated the size of the extruded preparation was determined by QELS (0). The size of streptavidin-liposome conjugates prepared as in figure 7 are graphed for comparison (0).

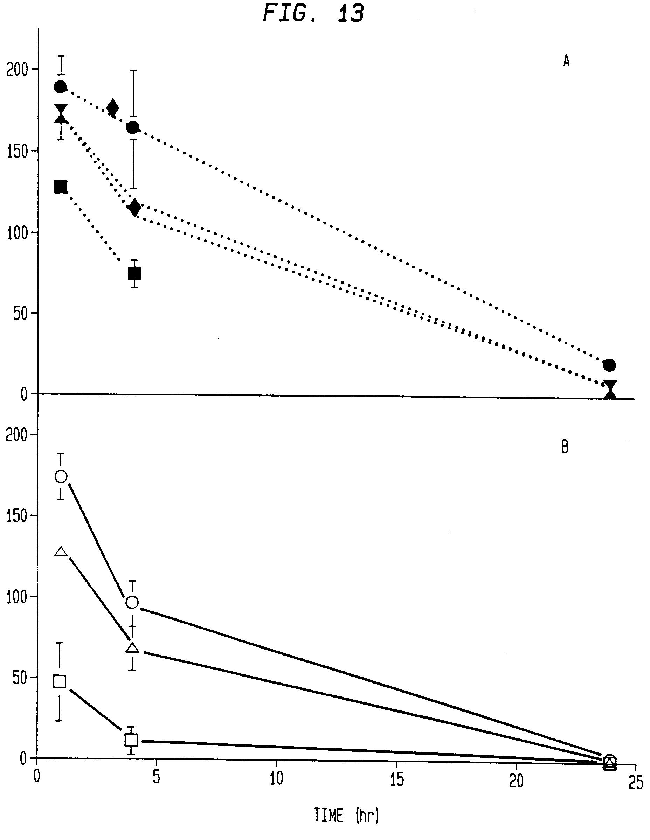

Figure 13 is representative of the in vivo clearance rates of liposome preparations as described in Example 23. Streptavidin was coupled to liposomes (50 and 100 nm) at a final lipid concentration of 30 mM and incubation period of 15 minutes, quenched with N-ethylmaleimide for 2 hours followed by an overnight incubation with B-mercaptoethanol. Control liposomes containing MPB-PE were titrated to pH 7.5 and exchanged on sephadex G-50 equilibrated with HBS. EPC/CHOL liposomes were made up in HBS. Mice (4-8 mice per time point) were injected with lipid at a dose of 100 mg per kg. Plasma was prepared from EDTA whole blood at specific time points and aliquots were analysed by scintillation counting as described in the materials and methods section of Example 13. The size of extruded samples was determined by QELS. (A): EPC/CHOL, 125 nm (closed circle); EPC/CHOL, 197 nm (closed square); MPB-PE liposomes, 170 nm (quenched closed inverted triangle, unquenched (closed triangle); (B): aggregated 100 nm streptavidin-liposomes, 530 nm (open square); streptavidin- liposomes extruded through 100 nm filters, 187 nm (open triangle); streptavidin-liposomes extruded through 50 nm, 139 nm (open circle).

### DETAILED DESCRIPTION OF THE INVENTION

The present invention relates to an improved method of synthesizing a substantially pure reactive lipid, for example, MPB-PE, which may be incorporated into liposomes, and subsequently reacted with a protein, antibody, cofactor or other molecule to produce a conjugated liposome. The conjugated proteins of the present invention may be utilized for numerous targeting applications including bioactive agent delivery as well as targeting for diagnostic uses.

In the method of the present invention, a liposome forming nucleophilic lipid, for example, phosphatidylethanolamine (PE) is reacted with a cross-linking agent, for example, N-succinimidyl 4-(p-maleimidophenylbutyrate (SMPB), to produce a substantially pure reactive lipid, for example, MPB-PE. In the method aspect of the present invention, the reaction of cross-linking agent and liposome forming nucleophilic lipid is performed in the presence of a non-nucleophilic solvent and optionally, a non-nucleophilic base, such as a tertiary amine. It has been discovered that the synthesis of MPB-PE by the prior art methods which utilize an alcoholic solvent in the presence of a non-nucleophilic base results in the production of substantial amounts of ring-opened side product. This ring-opened side product is significantly less reactive with thiol groups than is the maleimide group, resulting in the impure reactive lipid MPB PE of the prior art being less efficient for coupling proteins, antibodies cofactors and other molecules than is the MPB-PE of the present invention.

As used in the present invention, the cross-linking agents used to link conjugated molecules to reactive lipids are maleimide containing cross-linking agents, for example, those containing a p-maleimidophenylbutyrate group or other group, especially, for example, SMPB. Such agents are shown to be preferred reagents for cross-linking reactive liposomes and conjugated molecules, especially proteins. Although SMPB is a preferred reagent for use in the present invention, other agents containing maleimido groups may also be used. Among those cross-linking agents which may be used in the present invention include N-succinimidyl 3-maleimidobenzoate (SMB), N-succinimidyl 3-maleimidibutyrate (GMBS), N-succinimidyl 6-maleimidocaproate (EMCS), N-succinimidyl 3-maleimidopropionate, N-succinimidyl trans-4-(N-maleimidylmethyl) cyclohexane-1-carboxylate (SMCC) and N-succinimidyl maleimidylacetate (AMAS), among other maleimide containing cross-linking agents.

As used herein, the term liposome forming nucleophilic lipid refers to any lipid which may react with SMPB or an equivalent maleimide containing cross-linking agent to produce maleimido containing lipid or an equivalent lipid and which may be incorporated into liposomes with other liposome forming lipids. Such nucleophilic lipids include natural, synthetic and semisynthetic lipids such as phosphatidylethanolamine, dipalmitoylphosphatidylethanolamine DPPE), dimyristoylphosphatidylethanolamine (DMPE) and egg phosphatidylethanolamine (EPE), among others. In general, the more preferred nucleophilic lipids include those which contain an amine group, preferably a primary amine group, but other nucleophilic lipids including synthetic lipids containing alcoholic anions, such as oxy anions, among other nucleophilic groups, are also contemplated for use in the present invention. It will be recognized by those of ordinary skill in the art that the reactivities of the nucleophilic groups on the nucleophilic lipids and the amounts and concentrations of nucleophilic lipid and SMPB may be varied to produce pure reactive lipid. In general, it will be recognized that using more than a one-to-one molar ratio of nucleophilic lipid to SMPB may produce a maleimide ring-opened side product.

The reaction to produce reactive lipid proceeds in a non-nucleophilic solvent. As used herein, the term non-nucleophilic solvent refers to any solvent having favorable characteristics of dissolving the reactive components, including, for example, a non-nucleophilic amine, but which itself does not produce a ring-opened side product. Examples of solvents which may be used in the method aspect of the present invention include chloroform, methylene chloride and higher chlorinated and halogenated solvents, dimethylsulfoxide (DMSO), dimethylformamide (DMF), dimethylacetamide (DMA), 1, 4-dioxane, tetrahydrofuran (THF) and other ethers, among other solvents. Numerous other non-nucleophilic solvents may also be used in the present invention. It will be understood that the objectives in choosing a solvent for use in the present invention include maximizing the reaction to produce the reactive lipid and minimizing side products which are produced by the attack of solvent on the maleimide group of SMPB. It is to be recognized that the conditions of the prior art methods for producing reactive lipids are to be avoided and nucleophilic solvents such as methanol and ethanol, among other reactive alcohols, especially in the presence of base, are to be avoided.

It is important to note that in separating reactive lipid from displaced N-hydroxysuccinimide and other side products, the use of alcoholic and other nucleophilic solvents are to be avoided. Therefore, trituration or extraction techniques utilizing non-nucleophilic solvents are preferred for separating reactive lipids from more polar side products. As used herein, the term substantially pure reactive lipid refers to a reactive lipid in which the maleimido group is primarily intact, i.e., has not reacted with a nucleophile to produce a ring-opened side product. The term substantially pure reactive lipid should not be interpreted to exclude reactive lipid having substantially intact maleimide groups but which also may contain minor impurities and side products other than ring-opened side products.

The reactive liposomes of the present invention differ from prior art reactive liposomes in that the reactive liposomes of the present invention are substantially pure, i.e., they do not contain ring-opened reactive lipid, for example, PB-lipid. While not being limited by way of theory, such ring-opened reactive lipids are believed to affect the ability of the reactive liposome to form conjugated liposomes, and reactive liposomes containing appreciable amounts of ring-opened reactive lipids markedly reduce the efficiency of a liposome to conjugate a protein or other molecule. This reduced efficiency plus the fact that reactive lipids tend to destabilize conjugated liposomes results in the reactive lipids and liposomes produced therefrom having significantly more favorable characteristics, including enhanced stability as well as enhanced binding characteristics, than the prior art conjugated liposomes.

After the substantially pure reactive lipid is isolated, it is incorporated into liposomes to produce reactive liposomes, i.e., liposomes that can further react with conjugated molecules such as proteins, antibodies, cofactors and other molecules to produce conjugated liposomes. Generally, the reactive liposomes comprise at least about 0.05 mole percent of a reactive lipid and at least about 90% by weight of a liposome forming lipid. Preferably, however, the amount of reactive lipid is no greater than about 2.5 mole percent of the total weight of the reactive lipid to promote the stability of the reactive liposome. The reactive liposome may be formed using virtually any method available in the art for forming liposomes, with care being exercised to avoid disrupting the reactive moiety, for example MPB, which functions to covalently bind to protein, antibody or cofactor to produce conjugated liposomes.

In general, after the reactive liposome is formed, a protein or other group is then bound to the reactive liposome through the maleimido group on the reactive lipid. Any protein, antibody, cofactor or related molecule may be bound to the maleimido group provided that such molecule is sufficiently nucleophilic, preferably containing a thiol group. Present studies conducted evidence that the thiol group is clearly preferred over other nucleophilic groups, for example, amine groups or alcohol groups and is much more reactive than other groups in the presence of preferred conditions of pH and temperature of the coupling reaction.

It is clearly preferred to bind a conjugated molecule which has some targeting function, i.e., will bind to some active site, receptor site or other binding site for the purposes of delivering a bioactive agent or other molecule. Proteins which are useful in the present invention include streptavidin, various enzymes, immunomodulators, for example, interleukin-1, interleukin-2, tumor necrosis factor (TNF), various peptides and peptide fragments, especially those for use in vaccination, antibodies, for example, immunoglobulins such as IgG, IgM, IgE, monoclonal antibodies, and related proteins, among others. The present invention preferably contemplates the use of those proteins which covalently or non-covalently bind to the liposome and maintain their natural integrity so that, after binding to the reactive liposome, the protein may also bind to a target such as a receptor site, an antigenic determinant or other targeted binding site. Cofactors useful in the present invention especially include biotin because of its ability to non-covalently bind a number of proteins, including streptavidin and avidin.

Proteins useful in the present invention may be covalently linked to the reactive lipid of the reactive liposome, or alternatively, may be non-covalently linked to the reactive lipid through a cofactor, for example, biotin. Covalent linkages between the conjugated molecules and the reactive lipids may be formed by the reaction of thiol groups present in the protein or other molecule with the maleimido group of the reactive lipid. In cases where the conjugated molecule does not contain a thiol group, a thiol group may be introduced synthetically so that the molecule may be covalently linked to the liposome. In embodiments where the conjugated molecule is a protein, the protein may be modified with a bifunctional reagent, for example, N-succinimidyl 3-(2-pyridyldithiol) propionate (SPDP) to produce a protein containing two thiol groups which may react with the maleimido group of the reactive lipid.

Bifunctional reagents useful to modify a conjugated molecule for binding to the reactive lipid include those agents which contain at least one group which is reactive with the conjugated molecule and at least one group reactive with the maleimido group of the reactive lipid. A large number of bifunctional reagents are useful in the present invention as indicated hereinabove, for example, SPDP, succinimidylacetylthioacetate (SATA) and succinimidylacetylthioproprionate (SATP) among others.

In another aspect of the present invention, at the conjugated molecule, for example, protein may first be covalently linked to a cofactor , for example, biotin before the protein is covalently linked to the reactive lipid. In this aspect of the invention where biotin is employed, a protein such as streptavidin may be reacted with N-hydroxysuccinimide biotin or p-nitrophenyl biotin to produce a covalently biotinated protein for use in producing the protein-liposome conjugate.

In another aspect of the present invention, the protein-liposome conjugates containing streptavidin or other biotin-binding protein can be further coupled to proteins such as IgG or monoclonal antibodies which have been biotinated by coupling to biotin with, for example, N-hydroxysuccinimide biotin. Quite surprising is the observed stabililty of the protein-liposome conjugates which make the proteins an attractive coupler between the liposomes and the target sites.

In the aspect of the present invention in which protein is non-covalently bound to the reactive liposome, the liposomes are first formed utilizing most preferably between about 0.1 mole percent and about 1 mole percent of a reactive lipid, for example, phosphatidylethanolamine, covalently linked to a maleimide containing cross-linking agent which will react with a cofactor or modified cofactor. A preferred example of a cofactor to which certain proteins, for example, avidin and streptavidin will really bind is biotin. In certain aspects of the present invention where biotin is used, biotin may be introduced onto MPB-PE by modifying the biotin to contain a thiol group which then covalently binds to MPB-PE to produce a conjugated liposome containing biotin. A protein such as streptavidin may be non-covalently bound to the biotin of the conjugated liposome.

When protein is covalently or non-covalently linked to liposomes to produce protein-liposome conjugates, the liposomes may aggregate and increase in size. In such cases, it may be preferable to extrude the liposomes to produce sized liposome conjugates. Methods for producing sized liposomes to reduce aggregation are available in the art and have been previously described in United States Patent Application Serial Number 370,650, entitled "Preparation of Targeted Liposome Systems of a Defined Size Distribution", filed June 23, 1989 which is incorporated by reference herein.

In the method aspect of the present invention, the following steps are utilized:
1. SMPB is reacted with nucleophilic lipid in a non-nucleophilic solvent for a period of time sufficient to complete the conversion of nucleophilic lipid to reactive lipid;
2. After complete conversion of nucleophilic lipid to reactive lipid, the solution is concentrated in vacuo and the solid residue is triturated with solvent to remove unreacted SMPB and displaced succinimide to produce a substantially pure reactive lipid, for example, MPB-PE.

Preferably, an extraction step is employed in the reaction after step 1 to remove by-products such as N-hydroxysuccinimide. In that step, the reaction mixture from step 1 may be diluted with a non-nucleophilic solvent and then washed several times to remove by-products. After the extraction step is performed, step 2 above, the trituration step is generally performed.

One of ordinary skill in the art will recognize that various modifications of the above-identified reaction steps can be performed without departing from the invention of the present application, i.e., to produce substantially pure reactive lipid. For example, it will be recognized that instead of performing the trituration step (step 2, above), chromatographic separation employing non-nucleophilic solvents and conditions which avoid maleimide ring-opening could be performed.

The present invention also relates to reactive liposomes incorporating substantially pure reactive lipids of the present invention. The reactive liposomes of the present invention comprise at least one substantially pure reactive lipid, for example, MPB-PE, in an amount sufficient to bind conjugated molecules, in combination with at least one additional liposome forming lipid. Reactive liposomes of the present invention generally comprise at least about 0.05 mole percent of a substantially pure reactive lipid such as MPB-PE in combination with at least one liposome forming lipid in an amount generally no greater than about 99.95 mole percent of the reactive liposome. A discussion of liposome forming lipids which may be used in the reactive liposomes and conjugated liposomes of the present invention is detailed hereinbelow.

The present invention also relates to enhanced methods for coupling proteins onto MPB-lipid containing reactive liposomes. It has been found that the reaction to bind protein to reactive liposome is most efficient when conditions of pH 7.5 and temperatures of about room temperature, i.e., 23°C are employed. If one raises the pH or the temperature, the reaction will increase, but the integrity of MPB PE will decrease. Likewise if one lowers the temperature or the pH, the coupling of protein to reactive liposome will decrease, resulting in less efficiency. The conditions of the present invention, i.e., temperatures of about 23°C and a pH of about 7.5 are shown to produce the greatest efficiency of coupling of protein to reactive liposome to produce a protein conjugated liposome. In addition, the integrity of the reactive lipid is maximized under these conditions. Coupling efficiencies of over 50% are readily achieved under the optimized conditions of the method of the present invention. Similar efficiencies have been attained only on incorporation of higher levels of MPB-EPE in liposomes [5 mole %, see Bragman, et al., J. Natl. Cancer Inst., 73, 127, (1984)].

The present invention also relates to liposome conjugates which result from the coupling of the liposomes to conjugated molecules such as proteins, antibodies, cofactors and other molecules. Such liposomes comprise an amount of a reactive lipid effective to bind conjugated molecules, an amount of a liposome forming effective to form stable liposomes in combination with the reactive lipid and an amount of at least one conjugated molecule, for example, a protein, antibody, cofactor or other molecule, bound to the reactive liposome effective for targeting the liposome to a targeting site such as a receptor, active site or antigenic determinant.

The liposome conjugates of the present invention may be loaded with bioactive agent. In the case of liposome conjugates in which the conjugated molecule is a protein, such liposome conjugates may also be extruded to form sized liposomes to avoid aggregation of the liposomes. After the liposome conjugates of the present invention are formed, they may be dehydrated and rehydrated or alternatively, stored stably at 4°C. Alternatively, the liposome conjugates may be extruded to produce sized liposomes before they are loaded with a chosen bioactive agent by potential difference of ions across the bilayer membranes after formation, during the rehydration step or subsequently thereto. Preferred methods for loading bioactive agents into liposomes include those disclosed by Madden et al., in United States Application Serial No. 352,497, filed May 15, 1989, entitled "Accumulation of Drugs Into Liposomes by a Proton Gradient", relevant portions of which are incorporated by reference herein. Alternatively, the bioactive agent may be added to the liposome conjugates prior to dehydration.

The liposome conjugates of the present invention may be administered to a subject, for example, a mammal including humans. The composition may be delivered to such a subject parenterally in a pharmaceutically acceptable carrier or diluent such as phosphate buffered saline. The proteins bound to the liposomes aid in targeting the liposomes and their contents to a specific site in the body. When used parenterally, as in the case of bioactive agents such as antineoplastic agents, the amount used will be determined by the physician, and the treatment procedure as determined by the size of the tumor or other condition.

As described hereinabove, the present invention describes a method for producing sized protein-liposome conjugates. In the method of the present invention a liposome is first formed which comprises at least about 0.1 percent of a functionalized lipid and at least about 90 mole percent of a liposome producing lipid. Such a liposome is termed a reactive liposome. A reactive liposome is a liposome containing a functionalized lipid which will covalently or non-covalently bind to protein. After the reactive liposome is formed, a protein is coupled to the liposome to produce a protein-liposome conjugate. After the protein-liposome conjugate is formed, the conjugate is then extruded through a filter having a pore size ranging from about 30 nm to about 100 nm to produce sized protein-liposome conjugates. It has been found that the use of an extrusion step after the protein-liposome conjugate is formed results in non-aggregated protein-liposome conjugates of relatively small size, which are quite stable and which exhibit favorable blood circulation times. Surprisingly, during the extrusion step, the aggregated protein-liposome conjugates are not filtered out.

Any number of prior art methods may be utilized to covalently or non-covalently bind the protein to reactive liposomes to form the protein-liposome conjugates of the present invention. In general, however, the reactive liposomes are formulated to contain at least about 0.1 mole percent of a functionalized lipid, preferably no greater than about 10 mole percent and most preferably about 0.25 to about 1 mole percent of a functionalized lipid. As used throughout the specification of the present application, a functionalized lipid is any lipid which will form a liposome in combination with other liposome producing lipids and will bind (covalently or non-covalently) to a protein. A large number of functionalized lipids are contemplated by the present invention and are generally formed by reacting any one of a number of standard lipids used to form liposomes, for example, phosphatidylethanolamine (PE), with a bifunctional agent, for example, N-succinimidyl 4-(p-maleiimidophenyl) butyrate (SMPB) and N-succinimidyl 3-(2-pyridyldithiol) propionate (SPDP), N-succinimidyl trans-4-(N-maleimidylmethyl)cyclohexane-1-carboxylate (SMCC), and N-succinimidyl 3-maleimidylbenzoate (SMB) among others, to produce, for example the functionalized lipids MPB-PE and PDP-PE.

Functionalized lipids useful in the present invention are formed in two ways. The first way is by reacting a lipid with a bifunctional agent containing at least two functional groups; one of which covalently binds to the lipid and the other of which may be further reacted with a protein to produce a covalently linked protein-liposome conjugate. A bifunctional agent as used throughout the specification is a chemical agent which contains at least two distinct reactive groups which function to cross- link a lipid to a protein or a cofactor. Depending upon the chemistry of the functionalized lipid employed, the bifunctional reagent may contain at least two electrophilic groups such as activated esters, at least two nucleophilic groups such as amines, hydroxyls or thiols, or alternatively, at least one electrophilic group and one nucleophilic group. Of course, one of ordinary skill in the art would choose the bifunctional reagent to maximize the production of covalent linkages between the bifunctional reagent and the lipid or protein. Where needed, blocking groups, readily available in the art, are to be used to maximize the production of covalent linkages and prevent reaction between two different bifunctional reagents.

Alternatively, a functionalized lipid may also be formed by reacting a lipid, which contains a reactive group such as an amine or a hydroxyl group, for example, PE with an intermediate, for example, N-succinimidylbiotin or p-nitrophenylbiotin to introduce onto the lipid a cofactor or other group, for example, biotin, to which certain proteins readily non-covalently bind, to form biotin-PE. Functionalized lipids to which are bound cofactors, will non-covalently bind to a biotin-requiring protein such as streptavidin or avidin to produce non-covalently bound protein-liposome conjugates of the present invention.

The functionalized lipid is mixed with other traditional liposome producing lipids to produce reactive liposomes. The functionalized lipid generally comprises at least about 0.1 mole percent, preferably no greater than 10 mole percent and most preferably between about 0.25 and about 1 mole percent of the total lipid content of the reactive liposomes. While it is recognized that the amount of functionalized lipid which may be incorporated into liposomes may be greater than 10 mole percent, such an amount serves no useful function in the present invention.

In the general method of the present invention, after the liposome vesicle containing the functionalized lipid is formed, a protein is then bound to the reactive liposome through the functionalized lipid. Any protein may be bound to the liposome. However, the present invention preferably contemplates those proteins which covalently or non-covalently bind to the liposome and maintain their natural integrity so that, after banding to the reactive liposome, the protein may also bind to a target such as a receptor site, an antigenic determinant or other binding site. Proteins which are useful in the present invention include streptavidin, antibodies, for example immunoglobulins such as IgG, IgM, IgE, monoclonal antibodies, enzymes, immunomodulators, for example interleukin-1, interleukin-2, tumor necrosis factor (TNF) and peptides for use in vaccination, among others.

Proteins useful in the present invention may be covalently linked to the functionalized lipid of the liposome or alternatively, may be non-covalently linked to the functionalized lipid through, for example, a cofactor, such as biotin. Covalent linkages between the proteins and the functionalized lipid may be formed by the reaction of cysteinyl thiol groups naturally present within the protein with the functionalized lipid, or alternatively, the protein may be modified with a bifunctional reagent, for example, SPDP, to yield modified proteins having a reactive group, for example, a thiol which will react with the functionalized lipid. Where the protein to be covalently linked to the functionalized lipid contains at least two natural cysteinyl thiol groups which are relatively exposed, i.e., sufficiently exposed to the external surface of the protein to react with the functionalized lipid of the liposome without affecting the binding of the protein to a target site, there may be no need to modify the protein with a bifunctional reagent. However, where the protein to be covalently linked to the liposome contains no cysteinyl residues or the cysteinyl residues can only be exposed by disturbing the binding of the protein with a target, it may be necessary to functionalize the protein with a bifunctional reagent. The function of the protein bifunctional reagent is to covalently bind the protein to the functionalized lipid.

Bifunctional reagents useful in this aspect of the present invention include the same bifunctional reagents which may be used to bind to the lipid to produce a functionalized lipid. In this aspect of the present invention, the bifunctional reagent contains at least one group reactive with the protein and at least one group reactive with the functionalized lipid. A large number of bifunctional reagents are useful in the present invention, as indicated hereinabove. A particularly preferred bifunctional reagent useful in this aspect of the present invention is SPDP.

In another aspect of the present invention, the protein may first be covalently linked to a cofactor, for example, biotin before producing the protein-liposome conjugate. The protein-biotin composition may then be covalently linked to the functionalized lipid of the reactive liposome to produce a protein-liposome conjugate to which the cofactor is covalently bound. In this aspect of the invention where biotin is the cofactor employed, a protein such as streptavidin may be reacted with N-hydroxysuccinimide biotin or p-nitrophenyl biotin to produce a covalently biotinated protein for use in producing the protein-liposome conjugate.

In another aspect of the present invention, the protein-liposome conjugates containing streptavidin or other biotin-binding protein can be further coupled to proteins such as Immunoglobulin G or monoclonal antibodies which have been biotinated by coupling to biotin with, for example N-hydroxysuccinimide. Quite surprising is the observed stability of the protein-liposome conjugates which makes the proteins an attractive coupler between the liposomes and the target sites.

In the aspect of the present invention in which protein is non-covalently bound to the reactive liposome, the liposomes are first formed utilizing most preferably between about 0.1 mole percent and about 1 mole percent of a functionalized lipid, for example phosphatidylethanolamine, covalently linked to a cofactor, substrate or other molecule to which a protein will bind non-covalently. A preferred example of a cofactor to which certain proteins, for example, avidin and streptavidin will readily bind is biotin. In certain aspects of the present invention where biotin is used, biotin is introduced onto phosphatidylethanolamine to produce the functionalized lipid biotin-PE. The functionalized lipid is incorporated into a reactive liposome and a protein is non-covalently bound to the cofactor of the functionalized lipid. In certain aspects of the present invention the protein streptavidin is used to covalently bind to biotin of the functionalized lipid.

When protein is covalently or non-covalently linked to liposomes to produce protein-liposome conjugates, the liposomes tend to aggregate and increase in size. Without being bound by theory, it is believed that the aggregation phenomenon exhibited by protein-liposome conjugates may be the result of cross-linking that occurs between a protein containing more than one reactive group which links to functionalized lipids on more than one liposome, or alternatively, between a protein which non- covalently links cofactors on more than one liposome. In the case of non-covalent binding of streptavidin, the aggregation is believed to be the result of streptavidin being able to non-covalently bind to four biotin units on different liposomes. As a result of this cross-linking, the liposomes tend to aggregate or clump together, producing liposomes of greater size than the reactive liposomes to which the protein was bound. Based on the experiments performed, the amount of protein incorporated into the protein-liposome conjugate affects aggregation. As more protein is utilized the greater is the likelihood for cross-linking and aggregation and in general, the greater will be the size of the resultant protein-liposome conjugate.

The amount of protein utilized in the protein-liposome conjugate of the present invention ranges depending upon the size of the protein used, the strength of binding between the protein and a target site and the size of the liposome used. Generally, the protein is linked to the functionalized lipid in an amount equal to about 10 to about 100 protein molecules per liposome vesicle, and most preferably about 55 to about 80 protein molecules per liposome vesicle.

In the method of the present invention, it has been discovered that extruding the aggregated liposomes after attachment of the protein to the liposome reduces the size of the aggregated liposomes and produces smaller, stable, non-aggregated protein-liposome conjugates which exhibit significantly increased blood circulation times. By stable it is meant that the protein-liposome conjugates maintain the same approximate size and protein binding to a target for at least one hour and preferably at least four hours after extrusion. It is a surprising result that the protein-liposome conjugates are not filtered out during the extrusion process.

In the extrusion aspect of the present invention, aggregated protein-liposome conjugates are passed through filters having pore sizes generally ranging from about 30 nm to about 100 nm to produce protein-liposome conjugates ranging in size from about 75 to about 200 nm in diameter. Preferably, the pore size of the filters through which the protein-liposome conjugates are extruded ranges from about 50 nm to about 100 nm. The filters are generally made of polycarbonate, but the filters may be made of any durable material which does not interact with the protein-liposome conjugate and which is sufficiently strong to allow extrusion under sufficient pressure. Preferred filters include "straight through" filters because they generally can withstand the higher pressure of the preferred extrusion processes of the present invention. Although less preferred, "tortuous path" filters may also be used.

Any extrusion process available in the art may be used to produce the sized protein-liposome conjugates of the present invention and the extrusion of protein-liposome conjugates of the present invention may be performed sequentially or "straight through" under high pressure. Particularly preferred extrusion processes for use in the present invention include those disclosed in Cullis, et al., PCT Application PCT/US85/01161, Publication Number WO 86/00238 entitled "Extrusion Techniques for Producing Liposomes", published January 16, 1986, The present invention also relates to protein-liposome conjugates that result from the coupling of the liposomes to protein followed by an extrusion process. After the protein-liposome conjugates of the present invention are extruded, they may be dehydrated and rehydrated or alternatively, stored stably at 4°C. These compositions may be loaded with a chosen bioactive agent by potential difference of ions across the bilayer membranes after formation, during the rehydration step or subsequently thereto. Preferred methods for loading bioactive agents into liposomes include those disclosed by Madden, et al., in United States Application Serial Number 352,497, filed May 15, 1989, entitled "Accumulation of Drugs Into Liposomes by a Proton Gradient",. Alternatively, the bioactive agent may be added to the protein-liposome conjugates prior to dehydration.

The protein-liposome conjugates of the present invention may be administered to a subject, for example a mammal including humans. The composition may be delivered to such a subject parenterally in a pharmaceutically acceptable carrier or diluent such as phosphate buffered saline. The proteins bound to the liposomes aid in targeting the liposomes and their contents to a specific site in the body. When used parenterally as in the case of bioactive agents such as antineoplastic agents, the amount used will be determined by the physician, and the treatment procedure as determined by the size of the tumor or other condition.

The protein-liposome conjugates of this invention may also be used in diagnostic assays; in this case the amount of the protein-liposome conjugate used will depend on the sensitivity of the liposome-coupled antibody to the target components in the sample.

In certain preferred embodiments, the reactive liposomes used to form protein-liposome conjugates are themselves formed using the LUVET apparatus described in copending U.S. Patent Application entitled "Extrusion Technique for Producing Unilamellar Vesicles", Serial No. 622,690, filed June 20, 1984 and coupled to streptavidin using a modified technique of Leserman et.al., (Liposome Technology, III, 1984, CRC Press, Inc., N.Y., p. 29-40). Liposomes may be formed with a trans-membrane potential i.e., a Na+/K+ or H+ gradient across the bilayers, see copending U.S. Patent Application, Serial No. 749,161, Bally et.al., entitled "Encapsulation of Antineoplastic Agents in Liposomes", filed June 26, 1985, this potential difference is effected by the ionic concentrations of the internal versus the external media of the liposome. After loading the liposomes with bioactive agent, the liposomes are then dehydrated either in the presence or absence of sugars such as trehalose, and may be stored in this state for indefinite periods of time; see copending U.S. Patent Application, Serial No. 759,419, Janoff et.al., entitled "Dehydrated Liposomes," filed July 26, 1985

The reactive liposomes used in the present invention can have a variety of compositions and internal contents, and can be in the form of multilamellar, unilamellar, or other types of liposomes, or more generally, lipid-containing particles, now known or later developed. For example, the lipid-containing particles can be in the form of steroidal liposomes, stable plurilamellar liposomes (SPLVs), monophasic vesicles (MPVs), or lipid matrix carriers (LMC) of the types disclosed in commonly assigned U.S. Patent Applications Serial Nos. 476,496, 521,176, 591,576 and 599,691, filed March 24, 1983, August 8, 1983, March 20, 1984, and April 12, 1984, respectively However, it is to be recognized that the liposome should comprise at least about 0.1 mole percent and preferably no greater than about 10 mole percent of a functionalized lipid as herein defined.

Lipids which can be used in the liposome formulations of the present invention include synthetic or natural phospholipids and may include phosphatidylcholine (PC), phosphatidylethanolamine (PE), phosphatidylserine (PS), phosphatidylglycerol (PG), phosphatidic acid (PA), phosphatidylinositol(PI), sphingomyelin (SPM) and cardiolipin, among others, either alone or in combination. The phospholipids useful in the present invention may also include dimyristoylphosphatidylcholine (DMPC) and dimyristoylphosphatidylglycerol (DMPG). In other embodiments, distearylphosphatidylcholine (DSPC), dipalmitoylphosphatidylcholine (DPPC), or hydrogenated soy phosphatidylcholine (HSPC) may also be used. Dimyristoylphosphatidylcholine (DMPC) and diarachidonoylphosphatidylcholine (DAPC) may similarly be used. Due to the elevated transition temperatures (Tc) of lipids such as DSPC (Tc of about 65°C), DPPC (Tc of about 45°C) and DAPC (Tc of about 85°C), such lipids are preferably heated to about their Tc or temperatures slightly higher, e.g., up to about 5°C higher than the Tc, in order to make these liposomes. In preferred embodiments, egg phosphatidylcholine is used.

In a number of embodiments of the present invention, a steroidal component may be added to the liposome. For purposes of the present invention any component including the above-described phospholipids which may be used to produce a liposome either alone or in combination with a phospholipid is termed a liposome producing lipid. In preferred embodiments of the present invention, the liposome producing lipid comprises at least 90 mole percent of the total weight of lipids of the liposome. Any of the above-mentioned phospholipids may be used in combination with at least one additional component selected from the group consisting of cholesterol, cholestanol, coprostanol or cholestane. In addition, polyethylene glycol derivatives of cholesterol (PEG-cholesterols), as well as organic acid derivatives of sterols, for example cholesterol hemisuccinate (CHS) may also be used in combination with any of the above-mentioned phospholipids. Organic acid derivatives of alpha-tocopherol hemisuccinate, (THS) may also be used. CHS- and THS-containing liposomes and their tris salt forms may generally be prepared by any method known in the art for preparing liposomes containing sterols, so long as the resultant phospholipid-sterol mixture yields stable liposomes which may be cross-linked with protein. In particular, see the procedures of Janoff, et al., U.S. Patent No. 4,721,612, issued January 26, 1988, entitled "Steroidal Liposomes", and Janoff, et al., PCT Publication No. 87/02219, published April 23, 1987, entitled "Alpha Tocopherol-Based Vehicles" In preferred embodiments cholesterol is utilized in combination with EPC in a weight ratio of cholesterol to EPC of about 45:54.

Techniques used for producing large unilamellar liposomes (LUVs), such as, reverse-phase evaporation, infusion procedures, and detergent dilution, can be used to produce the reactive liposomes. A review of these and other methods for producing liposomes can be found in the text Liposomes, Marc J. Ostro, ed., Marcel Dekker, Inc., New York, 1983, Chapter 1

Several extrusion methods may be used to produce reactive liposomes or alternatively, protein-liposome conjugates. Preferably, to produce reactive liposomes, MLVs are extruded through filters forming large unilamellar vesicles (LUVs) of sizes dependent upon the filter size utilized. In general, polycarbonate filters of 30, 50, 60 or 100 nm pores may be used to produce the sized protein-liposome conjugates of the present invention. In this method, disclosed in Cullis, et al., PCT Publication Ho. WO 86/000238, January 16, 1986, the liposome suspension may be repeatedly passed through the extrusion device resulting in a population of liposomes of homogeneous size distribution. For example, the filtering may be performed through a straight-through membrane filter (a Nucleopore polycarbonate filter) or a tortuous path filter (e.g. a Nucleopore filter membrafil filter (mixed cellulose esters) of 0.1 um size), or by alternative size reduction techniques such as homogenization. Although the size of the reactive liposomes may vary from about 30 to above about 200 nm in diameter, preferably, the reactive liposomes are about 100 nm to about 200 nm in size. Generally, sized protein-liposome conjugates range in size between about 75 nm and about 200 nm.

As described hereinabove, a number of lipids may be used to form reactive liposomes having a gel to liquid crystalline Tc above ambient temperature. In such cases, an extruder having a heating barrel or thermojacket may be employed. Such a device serves to increase the liposome suspension temperature allowing extrusion of the LUVs. The lipids which are used with the thermojacketed extruder are, for example, DSPC, DPPC, DMPC and DAPC or mixtures thereof, which may include cholesterol in certain embodiments. Liposomes containing DSPC are generally extruded at about 65°C, DPPC at about 45°C and DAPC at about 85°C (about 5°C above the lipid Tc).

After extrusion, the reactive liposomes or protein-liposome conjugates may be loaded with bioactive agent or dehydrated for storage. However, in the case of protein-liposome conjugates, some loss of bioactive agent may result during the extrusion step. To avoid this possible result, it is preferred to load the bioactive agent after extrusion. The liposomes and protein-liposome conjugates of the present invention may be dehydrated using standard freeze-drying equipment or equivalent apparatus, and, if desired, the liposomes or protein-liposome conjugates and their surrounding medium can be frozen in liquid nitrogen before being dehydrated. Alternatively, the liposomes and protein-liposome conjugates can also be dehydrated without prior freezing, by simply being placed under reduced pressure. Dehydration with prior freezing requires the presence of one or more protective sugars in the preparation. A variety of sugars can be used, including such sugars as trehalose, maltose, sucrose, glucose, lactose, and dextran. In general, disaccharide sugars have been found to work better than monosaccharide sugars, with the disaccharide sugars trehalose and sucrose being most effective.

The one or more sugars are included as part of either the internal or external media of the liposomes or protein-liposome conjugates. Most preferably, the sugars are included in both the internal and external media so that they can interact with both the inside and outside surfaces of the liposomes' and protein-liposome conjugates' membranes. Inclusion in the internal medium is accomplished by adding the sugar or sugars to the solute which the liposomes are to encapsulate. Since in most cases this solute also forms the bathing medium for the finished liposomes, inclusion of the sugars in the solute also makes them part of the external medium. Of course, if an external medium other than the original solute is used, e.g., to create a trans-membrane potential (see below), the new external medium should also include one or more of the protective sugars.

In the case of dehydration without prior freezing, if the liposomes and protein-liposome conjugates being dehydrated have multiple lipid layers and if the dehydration is carried out to an end point where there is sufficient water left in the preparation so that a substantial portion of the membranes retain their integrity upon rehydration, the use of one or more protective sugars may be omitted. It has been found preferable if the preparation contains at the end of the dehydration process at least about 2%, and most preferably between about 2% and about 5%, of the original water present in the preparation prior to dehydration.

Once the liposomes or protein-liposome conjugates have been dehydrated, they can be stored for extended periods of time until they are to be used. When the dehydrated liposomes or protein-liposome conjugates are to be used, rehydration is accomplished by simply adding an aqueous solution, e.g., distilled water, to the liposomes or protein-liposome conjugates and allowing them to rehydrate.

As discussed hereinabove, the liposomes and protein-liposome conjugate preparation of the present invention may be loaded with ionizable pharmacological agents, for example antineoplastic agents, wherein a trans-membrane potential is created across the bilayers of the liposomes or protein-liposome conjugates and the antineoplastic agent is loaded into the liposomes by means of the trans-membrane potential. The trans-membrane potential is generated by creating a concentration gradient for one or more charged species (e.g., Na+, K+ and/or H+) across the liposome membranes. The concentration gradient is created by producing liposomes and protein-liposome conjugates having different internal and external media, i.e., internal and external media having different concentrations of one or more charged species.

Specifically, reactive liposomes used to produce the protein-liposome conjugates of the present invention are prepared which encapsulate a first medium having a first concentration of the one or more charged species. For a typical liposome preparation technique (see discussion above), this first medium will surround the liposomes as they are formed, and thus the liposomes' original external medium will have the same composition as the first medium. To create the concentration gradient, the original external medium is replaced by a new external medium having a different concentration of the one or more charged species. The replacement of the external medium can be accomplished by various techniques, such as, by passing the liposome preparation through a gel filtration column, e.g., a Sephadex column, which has been equilibrated with the new medium, or by centrifugation, dialysis, or related techniques.

In accordance with the invention, it has been found that this trans-membrane potential can be used to load ionizable antineoplastic agents into the liposomes or alternatively, into the sized protein-liposome conjugates. Specifically, once liposomes having a concentration gradient and thus a trans-membrane potential of the appropriate orientation have been prepared, the process of loading pharmaceutical agents into the liposomes reduces to the very simple step of adding the agent to the external medium. Once added, the trans-membrane potential will automatically load the agent into the liposomes.

The trans-membrane potential loading method can be used with essentially any pharmacological agent, including antineoplastic agents, which can exist in a charged state when dissolved in an appropriate aqueous medium (e.g., organic compounds which include an amino group which can be protonated). Preferably, the agent should be relatively lipophilic so that it will partition into the liposome membranes. Examples of some of the pharmacological agents which can be loaded into liposomes by this method include antineoplastic agents, for example, doxorubicin, mitomycin, bleomycin, daunorubicin, streptozocin, vinblastine, vincristine, mechlorethamine hydrochloride, melphalan, cyclophosphamide, triethylenethiophosphoramide, carmustine, lomustine, semustine, fluorouracil, hydroxyurea, thioguanine, cytarabine, floxuridine, decarbazine, cisplatin and procarbazine; local anaesthetics, for example, lidocaine, dibucaine and chlorpromazine; bronchodilators, for example, metaproterenol, terbutaline and isoproterenol; beta-adrenergic blockers, for example propanolol, timolol and labetolol; antihypertensive agents, for example clonidine and hydralazine; anti-depressants, for example, imipramine, amitryptyline and doxepim; anti-convulsants, for example, phenytoin; anti-emetics, for example, procainamide and prochlorperazine; antihistamines, for example, diphenhydramine, chlorpheniramine and promethazine; anti-arrhythmic agents, for example, quinidine and disopyramide; anti-malarial agents, for example, chloroquine, quinacrine and quinine; and analgesics, among a number of additional pharmaceutical agents.

In addition to loading a single pharmacological agent, the method can be used to load multiple pharmacological agents, either simultaneously or sequentially. Also, the protein- liposome conjugates into which the ionizable antineoplastic agents are loaded can themselves be pre-loaded with other antineoplastic agents or other drugs using conventional encapsulation techniques (e.g., by incorporating the drug in the buffer from which the liposomes are made).

It has been found that the rate of release of a pharmacological agent can be markedly reduced by creating a trans-membrane potential across the protein-liposome conjugate membranes which is oriented to retain the agent within the conjugate. That is, for an agent which is positively charged when ionized, a trans-membrane potential is created across the protein-liposome conjugate membranes which has an inside potential which is negative relative to the outside potential, while for an agent which is negatively charged, the opposite orientation is used.

As with the trans-membrane loading aspects of the invention, the trans-membrane potentials used to reduce the rate of drug release are created by adjusting the concentrations on the inside and outside of the liposomes or protein-liposome conjugates of a charged species such as Na+, K+ and/or H+. Indeed, if the liposomes or protein-liposome conjugates have been loaded by means of a trans-membrane potential produced by such a concentration gradient, simply keeping the liposomes or protein-liposome conjugates in an external medium which will maintain the original concentration gradient will produce the desired reduction in the rate of release. Alternatively, if a trans-membrane potential has not already been created across the liposome or protein-liposome conjugates membranes, e.g., if the liposomes or protein-liposome conjugates have been loaded using a conventional technique, the desired trans-membrane potential can be readily created by changing the composition of the external medium using the exchange techniques described above.

In the method aspect of the invention relating to dehydration of the protein-liposome conjugates, two basic approaches are provided. In the first approach, the conjugates can be loaded with bioactive agents (e.g., using conventional techniques or the trans-membrane potential loading technique described above), dehydrated for purposes of storage, shipping, and the like, and then rehydrated at the time of use. Alternatively, pre-formed liposome conjugates can be dehydrated for storage, etc., and then at or near the time of use, rehydrated and loaded with an ionizable bioactive agent using the trans-membrane potential loading technique described above.

When the dehydrated protein-liposome conjugates are to be used, rehydration is accomplished by simply adding an aqueous solution, e.g., distilled water or an appropriate buffer, to the protein-liposome conjugates and allowing them to rehydrate. The conjugates may be resuspended into the aqueous solution by gentle swirling of the solution. The rehydration can be performed at room temperature or at other temperatures appropriate to the composition of the liposomes and their internal contents.

If the bioactive agent which is to be administered is incorporated into the protein-liposome conjugates prior to dehydration, and no further composition changes are desired, the rehydrated conjugates can be used directly in therapy following known procedures for administering liposome encapsulated drugs.

Alternatively, using the trans-membrane potential procedures described above, ionizable bioactive agents can be incorporated into the rehydrated protein-liposome conjugates just prior to administration. In connection with this approach, the concentration gradient used to generate the trans-membrane potential can be created either before dehydration or after rehydration using the external medium exchange techniques described above.

Protein-liposome conjugates having the same internal and external media, i.e., no trans-membrane potential, can be prepared, dehydrated, stored, rehydrated, and then the external medium can be replaced with a new medium having a composition which will generate trans-membrane potentials, and the trans-membrane potentials used to load ionizable antineoplastic agents into the liposomes. Alternatively, protein-liposome conjugates having internal and external media which will produce trans-membrane potentials can be prepared, dehydrated, stored, rehydrated, and then loaded using the trans-membrane potentials.

Protein-liposome and liposome conjugates of the present invention may be administered to a subject such as a mammal, including humans. For administration to humans in the treatment of afflictions, the prescribing physician will ultimately determine the appropriate dose for a given human subject, and this can be expected to vary according to the age, weight, and response of the individual as well as the nature and severity of the patient's symptoms.

The mode of administration may determine the sites in the organism to which the compound will be delivered. For instance, delivery to a specific site of infection may be most easily accomplished by topical application (if the infection is external, e.g., on areas such as the eyes, skin, in the ears or on afflictions such as wound or burns) or by absorption through epithelial or mucocutaneous linings (e.g., nasal, oral, vaginal, rectal, gastrointestinal, mucosa, etc.). Such topical application may be in the form of creams or ointments. The protein-liposome conjugate containing bioactive agent may be administered alone but will generally be administered in admixture with a pharmaceutical carrier selected with regard to the intended route of administration and standard pharmaceutical practice. The protein-liposome conjugates of the present invention may be injected parenterally, for example, intravenously, intramuscularly, or subcutaneously. For parenteral administration, they are best used in the form of a sterile aqueous solution which may contain other solutes, for example, sufficient salts, glucose or dextrose to make the solution isotonic.

For the oral mode of administration, protein-liposome conjugate compositions of the present invention can be used in the form of tablets, capsules, lozenges, troches, powders, syrups, elixirs, aqueous solutions and suspension, and the like. In the case of tablets, carriers which can be used include lactose, sodium citrate, and salts of phosphoric acid. Various disintegrants such as starch, and lubricating agents, for example, starch may be used. For oral administration in capsule form, useful diluents are lactose and high molecular weight polyethylene glycols. When aqueous suspensions are required for oral use, certain sweetening and/or flavoring agents can be added.

The protein-liposome conjugates of the present invention may also be used in diagnostic assays; in this case the amount of the composition used will depend on the sensitivity of the liposome-coupled antibody to the target components in the sample.

The following examples are provided for purposes of illustration only and are not to be viewed as a limitation of the scope of the invention.

### EXAMPLES

### MATERIALS AND METHODS

Egg phosphatidylcholine ((EPC), egg phosphatidylethanolamine (EPE), and dipalmitoyal phosphatidylethanolamine (DPPE) were obtained from Avanti Polar Lipids (Birmingham, Ala. USA). N-succinimidyl 3-(2-pyridyldithio) propionate (SPDP), N-succinimidyl 4-(p-maleimidophenyl) butyrate (SMPB) were obtained from Molecular Probes, Oregon, USA and N-hydroxysuccinimide biotin (NHS-biotin) were obtained from Pierce Chemicals. Dithiothreitol (DTT), N-2-hydroxyethyl) piperazine-N'-3-propanesulphonic acid (EPPS), 2-(N-morpholino)-ethanesulphonic acid (MES), N-2-hydroxyethylpiperazine-N'-2-ethanesulphonic acid (HEPES), FITC-cellite, ethylene diamine tetra-acetate (EDTA), dithiobis-2-nitrobenzoic acid (DTNB), N-ethylmaleimide (NEM), bovine serum albumin (BSA), carboxyfluorescein, streptavidin, biotinated-protein A,, biotinated-alkaline phosphatase, biotinated-succinylated concanavalin A and sephadex G 50 were obtained from Sigma, USA. Anti-human erythrocyte IgG was purchased from Cappel, Inc. USA and biotinated anti-B 1 (PAN-B, IgG 2a) and biotinated anti-T 11 (E-rosette, IgG 1) were obtained from Coulter Electronics, USA. Sepharose CL-4B and ficoll paque were obtained from Pharmacia, Hew Jersey, USA. ³H biotin was obtained from Amersham, New Jersey USA and ¹⁴C cholesterol was obtained from New England Nuclear, USA.

### EXAMPLES 1 AND 2

### Synthesis of N-3[-(2-Pyridyldithio)propionyl-] phosphatidylethanolamine (PDP-PE) and N-[4-(p-Maleimidophenyl)butyryl] phosphatidylethanolamine (MBP-PE)

PDP-EPE was synthesized as described by Leserman, et al., Nature (London), 288, 602 (1984). Briefly, 50 umole of EPE wasdissolved in 3.5 ml chloroform/methanol (9:1) and added to 15 ml methanol containing 60 umole SPDP and 1000 umole triethylamine. After a 4 hour incubation at room temperature, analysis by thin layer chromatography (TLC, running solvent:chloroform/methanol/water, 65:25:4) indicated 99% conversion of EPE to a faster running product. The reaction mixture was washed with 10 ml of phosphate buffered saline. This washing was repeated three times prior to removal of the organic phase under reduced pressure. Analysis by two dimensional TLC and proton NMR indicated a single product which was greater than 98% pure. PDP-PE was stored under nitrogen in chloroform at 20°C for several months.

MBP-PE was initially synthesized according to the method of Martin, et al. (1982) with minor modifications. EPE (100 umole) was dissolved in 5 ml of anhydrous methanol containing 100 umole of freshly distilled triethylamine and 50 mg of SMPB. The reaction was carried out at room temperature under nitrogen and its progress monitored by TLC (running solvent:chloroform/methanol/water, 65:25:4). Following an 18 hour incubation, 95% of the EPE was converted to a faster running product. Methanol was removed under reduced pressure, the sample was dissolved in chloroform and washed extensively with 1% NaCl to remove unreacted SMPB and residual triethylamine. TLC analysis using the solvent system employed by Martin et al., J. Biol. Chem., 257, 286 (1982) indicated that the lipid product ran as a single component which was ninhydrin-insensitive and phosphate positive. Further characterization of the reaction products by 2-dimension TLC (first dimension, base: chloroform/methanol/25% Nh₃/H₂O, 90/54/5.7/5.3; second dimension, acid; chloroform/methanol/acetic acid/H₂O, 60/30/18/2..85) indicated the presence of two ninhydrin negative, phosphate positive lipid components (R_{f} values in acide dimension: 0.93 and 0.783). These observations were confirmed by ¹H NMR analysis and the slower running product, which comprised approximately 60% of the total lipid fraction, was identified as pure MPB-DPPE.

The two thiol reactive lipids synthesized above, PDP-EPE and MPB-EPE were subjected to coupling reactions with thiolated IgG according to prior art methods to determine which of the two cross-linking groups was the more efficient. As shown in Figure 1, significant coupling of thiolated IgG to liposomes containing PDP-EPE did not occur until greater than 20 mole % cholesterol was incorporated into liposomes. In contrast, levels of 12 ug IgG/umole lipid were obtained for liposomes containing MPB-EPE, even in the absence of cholesterol. The level of liposomally conjugated protein increased linearly with respect to amounts of cholesterol incorporated into vesicles. Significantly higher coupling ratios were obtained for the maleimide derivative of EPE under all conditions examined.

### EXAMPLE 3

### Synthesis of Pure MPB-PE

Pure MPB-DPPE was synthesized by reacting DPPE (69 mg) with SMPB (65 mg) in chloroform (5 ml) containing triethylamine (10 mg) at 40°C. After two hours, TLC on silica showed conversion of DPPE to a faster running product (solvent system: chloroform/methanol/acetonitrol/water, 75:16:5:4, Rf: 0.6). The solution was diluted with chloroform (10 ml) and washed several times with NaCl (0.9%) to remove by-products of the reaction. The solution was further concentrated in vacuo and the solid residue was triturated and recrystallized from diethylether to remove unreacted SMPB. Further recrystallization from diethylether/acetonitrile yielded a pure product as indicated by ¹H NMR analysis (Bruker W40, 400MHz). Fast Acting Bombardment (FAB) mass spectra were obtained at the British Columbia Regional Mass Spectroscopy Center, University of British Columbia, with AEI MS9.

### EXAMPLE 4

### Analysis of Reaction to Form MPB-PE

¹H NMR analysis of the lipid product obtained by reacting DPPE with SMPB according to Example 2 indicated the loss of the signal attributed to N-hydroxysuccinimide group of SMPB with the appearance of new peaks in the low field region of the ¹H NMR spectrum which were not characteristic of the expected product (delta: 7.58, 7.15 and 6.45, 6.22). In order to gain a better understanding of the conditions for the derivatization of DPPE with SMPB, 2-methoxyethylamine (CH₃O-CH₂CH₂-NH₂; Fig. 3, structure A) was selected as a model amine to react with SMPB.

The ¹H NMR spectrum of SMPB exhibits low filed resonances attributed to the aromatic protons of the phenyl group (chemical shift (delta):7.3 and vinyl protons (delta: 6.86), and high field resonances for methylenes of the N-hydroxysuccinimidyl group (NHS, dela: 2.86). When 2-methoxyethyleamine was incubated with SMPB under similar conditions described for the prior art synthesis of MPB-PE, two major products more polar than SMPB were detected by TLC (See Fig. 3 and Table 1, below). The less polar product was identified as the amide formed by the displacement of N-hydroxysuccinimide (NHS) from SMPB by ¹H NMR analysis due to the loss of a peak at delta 2.86 for the 4 methylene protons of the NHS group in SMPB and the appearance of new peaks at delta 3.35 (due to OCH₃) and delta 3.45 (due to O-CH₂CH₂; Figure 3, structure B). Analysis of the more polar product by ¹H NMR revealed a pattern which was consistent with the ring opening of the maleimide group due to methanolysis of the ring structure (Figure 3, Structure C). For example, as indicated in Table 1, the signals for the four aromatic protons appeared as two distinct doublets at delta 7.58 (d,J=8H, two protons) while the resonances for the two vinyl protons shifted upfield and appeared as two doublets at delta 6.22 and delta 6.45 (J=13Hz). The appearance of a sharp peak at delta 3.86 which was integrated for three protons, was interpreted to arise due to the addition of methanol to the maleimide group, resulting in opening of the ring moiety. These conclusions were further supported by mass spectrum analysis (structure B: molecular formula C₁₇H₂₀N₂O₄, molecular ion at 316; structure C: molecular formula C₁₈H₂₄N₂O₅, molecular ion at 348). Also ¹H NMR analysis of the MPB-DPPE lipid synthesized according to the prior art method of Martin, et al. indicated the presence of a mixture of pure and ring open MPB-DPPE derivates in the sample.

The susceptibility of the maleimide group to methanolic ring cleavage under basic conditions was confirmed by formation of a more polar product when a methanol solution of SMPB was treated with triethylamine (Table 1, below). As SMPB was found to be much more stable in chloroform, the derivatization of DPPE with SMPB was carried out in this solvent containing one equivalent of triethyleamine. The resulting lipid derivative was shown to be pure MPB-DPPE by ¹H NMR (Figure 2). Mass spectroscopic analysis (FAB) confirmed the purity of the lipid derivative by the presence of a molecular ion at 955 which corresponded to a molecular formula of C₅₁H₈₄O₁₁PNa for the sodium salt of MPB-DPPE.

**Table 1**

| Summary of 1H NMR Chemical Shifts for SMPB and SMPB derivates of 2-Methoxyethylamine (MEA) and DPPE | | | | | |
|---|---|---|---|---|---|
| SAMPLE | PHENYL PROTONS | | VINYL PROTONS | | METHYLENES |
| | Intact d7.3 | Cleaved d7.58,7.15 | Intact d6.86 | Cleaved d6.45,6.22 | of NHS Group d2.86 |
| SMPB | X | | X | | X |
| SMPB+ Methanol | | X | | X | |
| MPB-MEA | X | | X | | |
| MPB-MEA+ Methanol | | X | | X | |
| MPB-DPPE (pure) | X | | X | | |
| MPB-DPPE+ Methanol | | X | | X | |

### EXAMPLE 5

### Preparation of Liposomes

Large unilamellar vesicles (LUV's) were prepared as described by Hope, et al. (1985). Briefly, appropriate aliquots of lipid mixtures in chloroform were deposited in a tube and dried to a a lipid film under a stream of nitrogen followed by high vacuum for two hours. Normally lipid samples (50-54% EPC, 45% cholesterol, 1-5% reactive lipid prepared according to examples 1-3) were hydrated in 150mM NaCl, 25 mM HEPES, 25mM MES, pH 6.5 and extruded 10 times through 2 stacked 100 nm filters. Just prior to coupling experiments, samples were titrated to the appropriate pH with NaOH. For studies on the thiol dependence of the coupling procedure, liposomes containing 1% (for coupling) and 5% for maleimide reactivity pure MPB-DPPE were prepared at pH 6.5 as described above, titrated to pH 7.5 with NaOH and an aliquot was incubated with b-mercaptoethanol for 5 minutes at a molar ratio of 10 moles b-mercaptoethanol/mole of maleimide lipid. Liposomes were separated from free b-mercaptoethanol on sephadex G-50 equilibrated with 25 mM HEPES, 25 mM MES 150 mM NaCl, pH 7.5. The coupling efficiency potential and the reactivity of the maleimide group of quenched liposomes was compared to that of unquenched samples. Lipid was estimated either by the colorimetric assay of Fiske and Subbarow, J. Biol. Chem., 66, 325 (1925) or by trace amounts of ¹⁴C cholesterol present in the lipid mixture. This was performed by scintillation counting in a Packard Tri Carb liquid scintillation analyzer.

### EXAMPLE 6

### Assay for Maleimide Reactivity

Reactivity of the maleimide groups of MPB-PE lipids was estimated by the thiol binding of b-mercaptoethanol to lipid derivatives and back titration of unbound b--mercaptoethanol with Ellman's reagent, dithiobis-(2-nitrobenzoic acid) (DTNB) as described by Sedlack, et al., Anal. Biochem., 25, 192, (1968). Liposomes (5% MPB-DPPE, 50% EPC, 45% Cholesterol, 1 umole in 200 ul) were incubated with b-mercaptoethanol (100 ul of 1mM) at pH 8.2 (0.2 M Tris Cl, 20 mM EDTA, 1% Triton-X-100, pH 8.2, 1.6 ml) for 30 minutes at room temperature. DTNB (100 ul, 20 nM in methanol) was added and the absorbance was measured at 412 nm after 30 minutes. The requirement for protein associated thiol groups in the coupling procedure is illustrated in Table 2, below. Prior exposure of MPB-DPPE liposomes to b-mercaptoethanol resulted in a decrease in the extent of liposomally conjugated-streptavidin when quenched samples were compared to control MPB-DPPE. This was paralleled by a decrease in the detectable reactivity of the maleimide group of the lipid derivative. Furthermore, native streptavidin did not associate with liposomes containing the maleimide lipid.

**Table 2**

| SAMPLE | ug STREPTAVIDIN /umole LIPID | % MALEIMIDE REACTIVITY | |
|---|---|---|---|
| | 8 HOURS | 0 HOURS | 8 HOURS |
| MPB-DPPE Liposomes | 36.0 | 100 | 73 |
| b-mercaptoethanol treated MPB-DPPE liposomes | 2.5 | 11 | 0 |
| MPB-DPPE liposomes + unthiolated streptavidin | 0 | 100 | 77 |

Results: Liposomes (1 or 5% MPB-DPPE, 54-50% EPC, 45% cholesterol) were quenched with b-mercaptoethanol (10 molar excess to MPB-DPPE) for 5 minutes at pH 7.5, exchanged on sephadex G-50 equilibrated with HBS pH 7.5 and incubated with streptavidin or alone (pH 7.5 for eight hours at room temperature). After 8 hours incubation, the extent of streptavidin conjugated to liposomes and the reactivity of the maleimide group was determined for control (unquenched MPB-DPPE liposomes or unthiolated streptavidin) and quenched samples. As indicated, in certain cases such as streptavidin, as with other proteins, the presence of reactive thiols greatly facilitates the coupling of protein onto reactive liposomes. In fact, in the case of streptavidin, the presence of thiol groups appears to be a necessity.

Separately, MPB-PE lipid synthesized by the porio art method of martin, et al., supra, and MPB-PE synthesized by the method of the present invention were subjected to titration with b-mercaptoethanol at pH of 7.5. Liposomes (1 or 5% MPB-PE, 54-50% EPC, 45% cholesterol) were quenched with b-mercaptoethanol (10 molar excess to MPBV-PE) for 5 minutes at pH 7.5, exchanged on sephadex G-50 equilibrated with HBS pH 7.5 and incubated with streptavidin or alone. After 8 hours of incubation, the extent of streptavidin conjugated to liposomes and the reactivity of the maleimide group was determined for control (unquenched) and quenched samples (see above). Pure MPB-PE produced greater quenching with b-mercaptoethanol than did the MPB-PE produced by the prior art methods. The absence of a large difference in the amount of streptavidin bound is probably the result of steric interactions hindering the thiol groups in streptavidin from reacting with maleimide. The results of this experiment appear in Table 3, below.

**Table 3**

| Reactivity of MPB-PE with Mercaptoethanol pH 7.5 | | | | |
|---|---|---|---|---|
| SAMPLE | TREATMENT | ug Streptavidin umole Lipid | % Maleimide Reactivity | |
| | | 8 hrs | 0 Hrs | 8 Hrs |
| RING OPEN MPB-PE | Nothing | 33.4 | 100 | 71 |
| | b-mercap. | 17.5 | 43 | 18 |
| INTACT MPB-PE | Nothing | 36.0 | 100 | 73 |
| | b-mercap. | 2.5 | 11 | 0 |

### Example 7

### Preparation of Streptavidin and IgG for Coupling

In certain cases, as indicated above, in order to couple streptavidin protein to reactive liposomes containing MPB-PE, it is necessary to modify the protein to introduce reactive thiol groups.

Streptavidin (5mg/ml in 25mM HEPES, 150 mM NaCl, pH 7.5; HBS pH 7.5), was modified with the amine reactive reagent, SPDP according to the published procedures of Carlsson, et al., Biochem. J., 173, 723 (1978). Briefly, SPDP (25 mM in methanol) was incubated t a 10 molar ratio to streptavidin at room temperature for 30 minutes. Unreacted SPDP was removed by gel filtration on sephadex G-50 equilibrated with HBS pH 7.5. PDP-modified streptavidin was reduced with DTT (25 mM, 10 minutes). The thiolated product was isolated by gel exclusion on sephadex G-50 equilibrated with the relevant buffer and was immediately used in coupling experiments. The extent of modification of streptavidin was determined by estimating the concentration of the protein at 280 nm (extinction coefficient, E₂₈₀:2770) prior to the addition of dithiothreitol (DTT) and the 2-thiopyridone concentration at 343 nm (E₃₄₃:7550) 10 minutes after addition of DTT. In the case of IgG, after modification with SPDP as described for streptavidin, the protein was fluorescently labeled with FITC-cellite (50% weight of IgG, 20 minutes). Prior to the treatment of the protein with DTT, the sample was separated from unreacted reagents on sephadex G-50 equilibrated with an acetate buffer (100 mM NaCl, 100 mM sodium acetate, pH 5.0) to protect against the reduction of the intrinsic disulfides of the molecule. Bot protein preparations were modified to the same extent with SPDP (about 5-6 SPDP molecules per protein).

### EXAMPLE 8

### Coupling of Proteins to Liposomes

The coupling of proteins to liposomes was performed by incubating the reduced PDP-modified protein with liposomes containing PDP-PE, MPB-EPE or pure MPB-DPPE at a ratio of 100 ug protein/umole lipid (1 mM final concentration) at various pH values. Unassociated protein was removed by gel filtration on sepharose CL-4B equilibrated with HBS pH 7.5. The extent of coupling of streptavidin to liposomes was assayed by monitoring the binding of ³H biotin to streptavidin. Briefly, streptavidin-liposomes (0.25 umole lipid in 0.5 ml) were incubated with ³H biotin (3.85 nmoles in 25 ul, 15.4 nmoles/uCi) for 10 minutes and unbound biotin was removed by gel exclusion on sepharose CL-4B equilibrated with HBS pH 7.5. The extent of ³H binding to a streptavidin sample (100 ug) after gel exclusion on sephadex G-50, was used as a reference for the calculation of coupling ratios. For the determination of the extent of antibody coupled to liposomes, samples (200 ul) were dissolved in ethanol (1.8 ml) and the liposome associated fluorescence was correlated to a known quantity of fluorescein labeled antibody. Fluorescence was monitored at 520 nm using a SLM-aminco SPF-500C spectrofluoremeter with an excitation wavelength of 495.

### EXAMPLE 9

### Optimal Conditions for Coupling Thiolated Streptavidin to Liposomes Containing Pure MPB-DPPE

Optimal conditions for coupling thiolated streptavidin to liposomes containing pure MPB-DPPE were investigated. The results are presented in Figures 4 and 5. The pH dependence of the binding of thiolated streptavidin to MBP-DPPE liposomes and the stability of the maleimide function were initially established. As shown in Figure 4, the amount of liposomally conjugated protein increased rapidly at pH values greater than 7.0.However, incubation of liposomes containing pure MBP-DPPE at pH values of 7.0 and above resulted in a corresponding rapid degradation of the maleimide group of the derivatized lipid. At pH 7.5 after 18 hours of incubation, significant levels of streptavidin were coupled to liposomes (45%) with acceptable loss of maleimide reactivity (65% remaining). For this reason, a pH of 7.5 was chosen for further optimization of the coupling reaction.

In Figure 5, a time course relating streptavidin binding to liposomes and reactivity of the maleimide lipid is presented. The results indicate that optimal levels of streptavidin conjugated to liposomes (approximately 37 ug/umole of lipid) were obtain with minimal degradation of the maleimide group after an incubation period of 8 hours at pH 7.5 and at room temperature.

### EXAMPLE 10

### Applicability of Coupling to a Variety of Biotinated Proteins

To show the applicability of the general methodology of the present invention in attaching various types of targeting molecules to liposomes, the binding of a variety of biotinated proteins to streptavidin-liposomes was examined. As shown in Table 4, below, on incubation of various biotinated proteins with streptavidin conjugated liposomes, approximately 2 protein molecules bind for every 3 molecules of streptavidin. The extent of binding of biotinated proteins to streptavidin coupled vesicles is independent of the size of the biotinated protein (MW: 42,000 - 150,000 D.) Briefly, streptavidin liposomes with 45.2 ug protein bound/umole lipid were prepared as described in Example 8. Fluorescein labeled biotinated proteins were incubated with conjugated liposomes at a 2 fold molar excess to streptavidin for 10 minutes at pH 7.5. The extent of coupling of biotinated proteins to streptavidin liposomes was determined after gel exclusion of samples on sepharose CL-4B by measuring the levels of fluorescence associated with liposomes for protein and scintillation counting for lipid.

**Table 4**

| Binding of Biotinated Proteins to Streptavidin Liposomes | | | |
|---|---|---|---|
| PROTEIN | ug/mole LIPID | nmole/umole LIPID | Molar Ratio Protein-Streptavidin |
| Anti-human Erythrocyte IgG (mw: 150 kD) | 62.6 | 0.417 | 1:1.68 |
| Alkaline Phosphatase (mw: 140 kD) | 77.7 | 0.555 | 1:1.25 |
| Protein A (mw: 43 kD) | 20.3 | 0.482 | 1:1.46 |
| Succinylated Con. A 26.4 (mw: 55kD) | 0.480 | | 1:1.46 |

### EXAMPLE 11

### Binding of Biotinated Proteins to Streptavidin-Liposomes

Anti-erythrocyte IgG was biotinated according to the method of Bayer, et al., FEBS Lett., 68, 240 (1976). All biotinated proteins were fluorescently labeled with FITC-cellite as described above for IgG. Proteins were incubated at a two fold molar ratio to streptavidin coupled to liposomes for 10 minutes. Unassociated protein was removed by gel exclusion on sepharose CL-4B pre-equilibrated with HBS pH 7.5. The extent of liposome associated protein was determined as described above for the fluorescently labeled IgG. Background binding of all biotinated proteins was shown to be negligible.

### EXAMPLE 12

### In Vitro Targeting of Streptavidin Liposome Conjugates

Liposomes with entrapped carboxyfluorescein (15mM) were coupled to thiolated streptavidin as described above at pH 7.5 and a final lipid concentration of 2.5 mM. The coupling reaction was quenched with N-ethylmaleimide (500 molar ratio to streptavidin_ after 4 hours, streptavidin liposome conjugates were isolated by gel exclusion on sepharose CL-4B and levels of liposomally associated streptavidin were determined as described above.

For targeting experiments, human blood was collected in EDTA (25 mM in PBS). Human peripheral blood leukocytes were isolated by standard protocols using Ficoll paque [see, Boyum, Scand. J. Clin. Lab. Invest., 21, Supp. 97, 9 (1968)] and suspended in PBS containing 2% BSA and 0.01% Na azide at 4°C prior to binding studies. Cells (10⁶) were aliquoted into round bottom microtitre wells, washed and incubated with antibody (T11 and B1, 5 and 10 ug respectively in 100 ul PBS) or alone in PBS for 1 hour at 4°C. After washing twice with PBS, cells were incubated with streptavidin liposome conjugates (0.2 umoles in 200 ul PBS) for a further hour at 4°C. The cells were then washed three times with PBS and analyzed by flow cytometry according to the procedure described below.

Briefly, cell associated fluorescence was measured with an EPICS Profile analyzer (Coulter Electronics, Inc.). Cells were illuminated with the 488 nm line of an argon ion laser. Fluorescence was measured behind a 515 to 530 nm band-pass filter. Fluorescence signals were gated on the basis of a right angle versus forward light scatter cytogram to restrict analysis to signals from single cells. Amplifiers were set in the log area mode. For statistical analysis of histograms, region 1 was arbitrarily set (min.: 2.705, max.: 1023) with the lower channel at the base of the right shoulder of the histogram of the control sample.

As shown in Figure 6, incubation of liposome streptavidin conjugates (containing encapsulated carboxyfluorescein) with cells pre-labeled with a biotinated monoclonal antibody specific for peripheral B cells (B1), resulted in the fluorescein labeling of approximately 20% of the total lymphocyte population (Figure 6B). In comparison, similar studies with a biotinated anti T cell antibody (T11) resulted in the labeling of approximately 90% of lymphocytes (Figure 6C). These results are consistent with the expected cell distribution of the antigens defined by T11 [See Howard, et al., J. Immunol., 126, 2117 (1981)] and B1 [See Stashenko, et al., J. Imnunol., 125, 1678 (1980)]. The specificity of these conjugates is indicated by, the negligible background binding of streptavidin liposome conjugates to lymphocytes in the absence of biotinated antibodies (Figure 6A).

### EXAMPLE 13

Egg phosphatidylcholine (EPC), and dipalmitoyl phosphatidylethanolamine (DPPE) were obtained from Avanti Polar Lipids USA. Biotin-phosphatidylethanolamine (biotin-PE), N-succinimidyl 3-(2-pyridyldithio) propionate (SPDP), N-succinimidyl 4-(p-maleiimidophenyl) butyrate (SMPB) were obtained from Molecular Probes, Oregon, USA. Streptavidin, FITC-cellite, N-ethylmaleimide, dithiothreitol, cholesterol, B-mercaptoethanol, N-(2-hydroxyethyl) piperazine-N'-3-propanesulphonic acid (EPPS), 2-(N-Morpholino) ethanesulphonic acid (MES), N-2-Hydroxyethylpiperazine-N'-2-ethanesulphonic acid (HEPES) and sephadex G-50 were obtained from Sigma, USA. Anti-human erythrocyte IgG was obtained from Cappel, Inc. USA and Sepharose CL-4B from Pharmacia, Canada. 14C cholesterol and 3H cholesterol-hexadecyl-ether were obtained from New England Nuclear, Canada. 3H and 14C biotin were obtained from Amersham, Canada. Mice, averaging 21 g. in weight, were obtained from Jackson Laboratories, California, U.S.A.

Synthesis of N-(4-(p-Maleimidophenyl)butyryl)dipalmitoylphosphatidyl-ethanolamine (MPB-DPPE) MPB-DPPE was synthesized by a modification of the method of Martin, et al., J.Biol.Chem., 257, 286-288, (1982). Briefly, synthesis of intact MPB-DPPE was carried out in the presence of one equivalent of triethylamine in chloroform, at a molar ratio of 1.5 SMPB:DPPE. After 3 hours, the reaction mixture was evaporated to dryness under nitrogen. Excess unreacted SMPB and major by-products were removed by preparative thin layer chromography (TLC, silica gel developed with 50% acetone in chloroform). The upper portion of the lipid band was extracted from silica with about 20 to 30% methanol in chloroform (V:V) resulting in the isolation of pure intact MPB-DPPE as characterized by 1H NMR.

### EXAMPLE 14

### Preparation of Liposomes

Large unilamellar liposomes were prepared as described by Hope, et al. Biochim. Biophys. Acta., 812, 55 (1985). Briefly, aliquots of lipid mixtures in chloroform were deposited in a tube and dried to a lipid film under a stream of nitrogen followed by high vacuum for 2 hours. Lipid was then hydrated in 25 mM MES, 25 mM HEPES, 150 mM NaCl pH 6.5 and extruded through two stacked 100 nm or 50 nm filters 10 times. Prior to coupling experiments, samples were titrated to pH 7.5 with NaOH. Lipid was estimated either by the colorimetric method of Fiske, C. and SubbaRow, Y., J. Biol. Chem., 66, 375 (1925) or by incorporating trace amounts of 14C cholesterol or 3H cholesterol-hexadecyl ether in the lipid mixture. The samples were assayed by scintillation counting in a Packard Tri Carb liquid or a Beckman model LS 3801 scintillation analyzer.

### EXAMPLE 15

### Preparation of Proteins for Coupling

Streptavidin (10 mg/ml in 25 mM HEPES, 150 mM NaCl, pH 7.5, HBS) was modified with the amine reactive reagent, SPDP according the procedure of Carlsson, et al., Biochem. J., 173, 723 (1978). Briefly, SPDP (25 mM in methanol) was incubated at a 10 molar ration to streptavidin at room temperature for 30 minutes. To estimate the extent of modification, a portion of the reaction mixture was passed down sephadex G-50 equilibrated with HBS to remove unreacted SPDP. The extent of modification of streptavidin was determined by estimating the protein concentration at 280 nm [Extinction coefficient at 280 nm (E280:2770)] prior to the addition of dithiothreitol (DTT) and the 2-thiopyridone concentration at 343 nm (E343:7550) 10 minutes after the addition of DTT (25 mM). The remainder of the reaction mixture was reduced with DTT (25 mM, 10 minutes) and the thiolated product was isolated by gel exclusion on sephadex G-50 equilibrated with 25 mM MES, 25 mM HEPES, 150 mM NaCl, pH 7.5. The product was immediately used in coupling experiments

In the case of IgG (20 mg/ml in HBS), following the modification of the protein with SPDP, the protein was fluorescently labeled with FITC-cellite (50% weight of IgG in 150 mM NaCl, 0.2 M NaHCO3, pH 8.8, 20 minutes). Prior to the treatment of the protein with DTT, the sample was separated from unreacted reagents on sephadex G-50 equilibrated with an acetate buffer (100 mM NaCl, 100 mM Na acetate, pH 5.0), to protect against the reduction of the intrinsic disulfides of the molecule. The sample was concentrated to 5 mg/ml by dehydration with aquacide prior to the coupling experiments. The extent of modification of streptavidin was 5-6 SPDP molecules per protein while the modification of the antibody preparation resulted in 2-3 molecules of SPDP per protein.

### EXAMPLE 16

### Covalent Coupling of Proteins to Liposomes

The coupling of proteins to liposomes was performed by incubating the reduced PDP-modified protein with liposomes (54% EPC, 45% cholesterol, 1% MPB-PE, sized through filters of 50 or 100 nm pore size), at a ratio of 100 ug protein/umole lipid (5 mM-30 mM final lipid concentration) at pH 7.5. The reaction was quenched at various times by the addition of N-ethylmaleimide (500 molar ratio to protein, in methanol). For in vivo experiments, samples were further quenched with B-mercaptoethanol (10 molar ratio with respect to N-ethylmaleimide) after a 2 hour incubation of the reaction mixture with N-ethylmaleimide. Uncoupled protein was removed by gel filtration on sepharose CL-4B equilibrated with HBS. The extent of coupling of streptavidin to liposomes was measured by the binding of 3H or 14C biotin to streptavidin. Briefly, streptavidin-liposomes (0.25 umoles lipid in 0.5 ml) were incubated with 3H or 14C biotin (3.85 nmoles in 25 ul, 15.4 nmoles/uCi) for 10 minutes and unbound biotin was removed by gel filtration on sepharose CL-4B equilibrated with HBS. The extent of binding of biotin to a streptavidin standard (100 ug) after gel exclusion on sephadex G-50 was used as a reference for the calculation of coupling ratios. For the determination of the extent of antibody (IgG) coupled to liposomes, samples (200 ul) were dissolved in ethanol (1.8 ml) and the liposomes associated fluorescence was correlated to a known quantity of fluorescein labeled antibody. Fluorescence was monitored at 520 nm using a SLC-500C spectrofluorometer with an extinction wavelength of 495 nm.

### EXAMPLE 17

### Preparation of Non-covalently Attached Streptavidin

Prior to the non-covalent attachment of streptavidin to liposomes, streptavidin was fluorescently labeled with FITC-cellite as described above for IgG. Streptavidin (4.1 mg) was incubated for 10 minutes with liposomes (54.75% EPC, 45% cholesterol, 0.25% biotin-PE) at a 10 molar excess to to biotin-PE in 20 mM Hepes-buffered saline (pH 8) for about 30 minutes. See Loughery, et al., Biochem. Biophys. Acta., 901, 157 (1987). At various times, aliquots were fractionated on Sepharose CL-4B columns (5 ml) to separate liposomally bound streptavidin from free streptavidin. The extent of coupled streptavidin was determined after gel filtration on sepharose CL-4B as described for IgG (Example 16, above).

### EXAMPLE 18

### Preparation and Characterisation of Extruded Protein-Liposome Samples

Protein-liposome conjugates (5 mM or 20 mM final lipid concentration) were extruded 10 times through two stacked millipore filters (50 or 100 nm). Lipid recovery was estimated by scintillation counting of an aliquot of the extruded sample. The size of the protein-coupled vesicles before and after extrusion was estimated by freeze fracture techniques and by quasi-elastic light scattering (QELS) using a Nicomp Model 270 submicroparticle size operating at 632.8 nm and 5 mW.

### EXAMPLE 19

### In Vivo Studies of Liposome Preparations

For in vivo studies, streptavidin-liposome-conjugates were prepared at a final lipid concentration of 30 mM and an incubation period of 15 minutes, as described in Examples 13 through 18. Liposomal lipid was quantified employing the non-metabolizable, non-exchangeable lipid marker 3H cholesterol-hexadecyl-ether by the method described in Huang, L., in "Liposomes", Ed. Mark J. Ostro, pp. 87-124, by Marcel Dekker, New York, 1983 and Stein, et al., FEBS Lett., 11, 104 (1980), specific activity: 0.23 uCi/mg total lipid. For scintillation counting, 50-100 ul plasma was added to 5 ml Pico0Fluor 40 (Packard, Canada) scintillation cocktail and samples were counted in a Beckman model LS 3801 scintillation counter. Unbound streptavidin was removed by gel exclusion on sepharose CL-4B. A portion of the sample was extruded 10 times through two stacked 50 or 100 nm filters immediately prior to injection. As controls, liposomes containing MPB-PE (54% EPC, 45% cholesterol, 1% MPB-PE) were prepared at pH 6.5 as described hereinabove. An aliquot of the lipid sample was titrated to pH 7.5 with NaOH, quenched with B-mercaptoethanol (10 molar excess to MPB-PE) and free B-mercaptoethanol was removed by gel filtration on sephadex G-50 equilibrated with HBS. Unquenched MPB-PE liposomes were exchanged on sephadex G-50 equilibrated with HBS prior to in vivo experiments. Liposomes containing 55% EPC and 45% cholesterol were prepared in HBS.

For in vivo plasma lipid level determinations, mice (4-8 per time point) were injected with samples via the tail vein at a dose of 100 mg total lipid/kg. Blood was collected in EDTA treated microcontainers (Bectin Dickinson, Franklin Lakes, New Jersey) and plasma was prepared by centrifuging (200 X g) whole blood for 10 minutes in a clinical centrifuge. Total plasma volume per animal was taken to be 4.55% of mean body weight. Control blood samples containing known amounts of liposomes showed that only a minor fraction of the liposomal lipid was associated with the pelleted blood cells. The recovery of liposomes was similar if determined from whole blood or from plasma. The levels of streptavidin associated with liposomes in vivo was determined by the binding of 14C biotin to a plasma sample isolated 1 and 4 hours post injection.

### EXAMPLE 20

### Characterization of Protein Conjugation on Vesicle Size

Liposomes (54%EPC, 45%CHOL, 1% MPB-PE, 5 mM final lipid concentration, 100 nm) were incubated with streptavidin (100 ug protein/umole lipid) over time at pH 7.5 as described hereinabove. At various times ranging from 5 minutes to 12 hours, as depicted in figure 1, the reaction was quenched by addition of N-ethylmaleimide (500 molar ratio to protein) and free streptavidin was removed by gel filtration on sepharose CL-4B. The extent of coupled streptavidin was determined by 3H binding (indicated in graph A of figure 1) and vesicle size was estimated by QELS (indicated in graph B of figure 1). As shown in figure 1, an increase in the amount of protein bound to liposomes results in a significant increase in vesicle size as recorded by QELS. The initial rapid coupling of streptavidin to vesicles correlates with a rapid increase in the size distribution of the preparation. In order to confirm this result, a freeze fracture technique for examining the morphology of the larger systems, was used to measure aliquots of the same coupling system. The results presented in Figure 2, clearly show that the increase in size as measured by QELS is related to vesicle aggregation. However, after extended periods of incubation, a significant number of large vesicles (> 200 nm) are observed, presumably due to fusion events following aggregation.

### EXAMPLE 21

### Effect of Extrusion on Aggregation of Liposome Conjugates

In an attempt to achieve small, homogeneously sized protein-liposome conjugates, the effects of extruding aggregated, conjugated vesicles through filters with 100 nm pore size were examined for liposomes with attached streptavidin (Figure 3) or antibody (Figure 4) as prepared hereinabove. The coupling reaction mixtures were quenched with N-ethylmaleimide at various times and the size of the coupled samples prior to and after extrusion was estimated by QELS (Figures 3B and 4B). The extent of coupled protein was determined after extrusion of conjugated samples (Figures 3A and 4A). Irrespective of the amount of protein coupled to the liposomes, vesicles coupled with streptavidin or antibody were readily extruded and the resulting preparations fell within a narrow size range. For example, extrusion of liposomes with attached streptavidin (25-60 ug/umole lipid) resulted in vesicle sizes of 120-140 nm in diameter as compared to initial size distributions of 150 to more than 500 nm.

Similarly, extrusion of antibody liposome conjugates (15-35 ug protein/umole lipid) resulted in smaller vesicles of narrow size distribution (90-110 nm) compared to the size range of 130-230 nm prior to extrusion. It is important to note that the loss of lipid for both types of protein coupled vesicles during the extrusion process was minimal (85-90% lipid recovery). These results demonstrate that a highly aggregated preparation of vesicles with high levels of conjugated protein can be extruded efficiently and the resulting preparations are of a similar size.

Furthermore, the extrusion of protein-liposome aggregates represents a gentle method preparing sized protein conjugated vesicles. This was illustrated by the retention of streptavidin-liposome conjugates to bind biotin after extrusion (results not shown).

The observation that liposome conjugates aggregate during protein coupling to liposomes is not unique to the covalent attachment of proteins to liposomes. Vesicle aggregation also occurs during the non-covalent attachment of streptavidin to liposomes contain- ing biotin-PE [See Loughery, et al., Biochem. Biophys. Acta., 901, 157 (1987)]. To demonstrate the general application of the extrusion process as a means of generating sized populations of protein-liposome conjugates, the effect of extrusion of streptavidin coupled covalently to liposomes containing MPB-PE or non-covalently bound to liposomes containing biotin-PE was examined by freeze fracture (Figure 5). Both types of streptavidin liposome conjugates were observed to be highly aggregated prior to extrusion. After extrusion, the coupled vesicles existed as monomers of dimers with the maximum aggregate observed to be a conglomerate of 4 vesicles. In the case of the non-covalent coupling procedure (Figure 5C and 5D), significant loss of lipid occurred (50%) during the extrusion of coupled vesicles.

### EXAMPLE 22

### The Stability of Extruded Liposomes

The stability of extruded samples containing covalently bound streptavidin with respect to size is represented by figure 6. QELS measurements indicate an initial small (30nm) rapid increase in the size of the preparation after extrusion. This was reflected by increased aggregation of the extruded vesicles as indicated by freeze fracture (results not shown). As shown in Table 5, below, the level of reaggregation observed 8 hours after extrusion of various streptavidin-liposome conjugates was minimal when compared to the aggregated state of the samples prior to extrusion. Reaggregation of liposomes was not observed when MPB-PE liposomes were extruded with thiolated-streptavidin which had been quenched by prior incubation with B-mercaptoethanol (Table 5, below). This indicates that reaggregation was not due to non-specific association of protein with liposomes. It was found that the incorporation of negatively charged lipids, for example phosphatidylserine, or the presence of low or high ionic strength buffers did not prevent reaggregation (data not shown). The reduction of the amount of streptavidin coupled to vesicles (Table 5, below) resulted in a corresponding decrease in the extent of reaggregation 8 hours after extrusion. Varying the lipid concentration of the extruded sample did not significantly affect the reaggregation. Streptavidin coupled to liposomes which were frozen immediately after extrusion, maintained their original size distribution on thawing. Finally, storage of the extruded samples at 4°C resulted in increased stability of liposome size.

**Table 5**

| Factors Affecting the Aggregation of Extruded Streptavidin-Liposomes QELS Size Estimates of Streptavidin Coupled to Liposomes (nm) | | | | |
|---|---|---|---|---|
| ug. Streptavidin/umol. Lipid | Lipid Conc.(mM) | Before Extrusion | After Extrusion | |
| | | | 0 Hrs | 8 Hrs |
| 0^{c} | 2.5 | 110 | 104 | 104 |
| 17.1^{a} | 2.5 | 177 | 109 | 119 |
| 31.6^{a} | 2.5 | 232 | 119 | 140 |
| 45.3^{a} | 2.5 | 286 | 123 | 154 |
| 45.1^{b} | 5.0 | 403 | 174 | 197 |
| 45.1^{b} | 15.0 | 403 | 174 | 197 |
| 45.1^{b}^{,}^{d} | 5.0 | 403 | 174 | 182 |
| 45.1^{b}^{,}^{d} | 5.0 | 403 | 174 | 188 |

| | | | | |
|---|---|---|---|---|
| ^{a} Liposome samples (54% EPC, 45% CHOL, 1% MPE-DPPE) were prepared with different levels of coupled streptavidin by quenching the coupling mixture (20 mM final lipid concentration) with N-ethylmaleimide at various times. | | | | |
| ^{b} Streptavidin-liposomes were prepared at a final lipid concentration of 30 mM and an incubation period of 15 minutes. | | | | |
| ^{c} Streptavidin (50ug) quenched with N-ethylmaleimide was extruded with liposomes (1 umole, 2.5 mM final lipid concentration) containing 1% MPB-DPPE. | | | | |
| ^{d} Extruded samples were kept on ice for 3 hours prior to QEL measurements. | | | | |
| ^{e} Extruded samples were frozen immediately after extrusion and thawed just prior to QEL measurements. | | | | |

### EXAMPLE 23

### Blood Clearance of Protein-Liposome Conjugates

Studies have shown that large liposomes are rapidly removed from the blood circulation when compared to small liposomes [See Hunt, A.C., Biochim. Biophys. Acta., 719, 450 (1982) and Sota, et al., Chem. Pharm. Bull., 34, 4244 (1986)]. Rapid clearance which was observed for targeted systems in vivo [See Wolff, et al., Biochim. Biophys. Acta., 802, 259 (1984) and Papahadjopoulos, et al., in "Annals of the New York Academy of Sciences", ed. R.L. Juliano, 507, 4035 (1988)] could partly be due to the aggregation of liposomes. To test this hypothesis, the time required for clearance from the blood of certain control liposome preparations (Figure 7A) as well as aggregated and extruded streptavidin-liposome conjugates (Figure 7B) in mice were therefore examined. Aggregated streptavidin-liposomes (530 nm in diameter as indicated by QELS) were cleared rapidly from the circulation; only 3% of the initial lipid dose remained in the circulation 4 hours after injection. Extrusion of these protein-vesicle conjugates through 50 or 100 nm polycarbonate filters resulted in preparations with size distributions of 139 and 187 nm respectively. Both of these preparations showed extended blood circulation times in vivo, with 48 and 32% of the initial dose remaining in circulation after 4 hours. When compared to EPC/CHOL vesicles of 125 nm size, the presence of covalently bound protein on liposomes of similar size (139 nm) enhanced the clearance of liposomes from the circulation (80 and 48% of EPC/CHOl vesicles remained in circulation after 4 hours versus 48 and 32% of protein-liposome conjugates). No significant difference in the circulation of MPB-PE liposomes (normal or quenched with B-mercaptoethanol, 170 nm in diameter) was observed when compared to EPC/CHOL preparations of 197 nm in diameter.

As shown here, the extent of aggregation of the coupled liposomes significantly alters the blood clearance behavior of the conjugated preparations. As indicated, the aggregated streptavidinliposomes (>530 nm in diameter) were rapidly removed from the circulation (<3% remaining after 4 hours). In comparison, extended circulation times were obtained for extruded conjugates i.e., 32 and 48% of the initial lipid dose remained in circulation 4 hours post-injection for samples of 187 nm and 139 nm in diameter, respectively. The enhanced circulation times observed for smaller protein-liposome conjugates indicates that aggregation of the preparation is a major factor that determines the lifetimes of conjugates in vivo. It should be noted, however, that the clearance of protein-liposome conjugates from the blood was always greater than for control samples of similar size, indicating that the presence of protein on liposomes contributes to some extent to an enhanced clearance of liposomes from the circulation. The presence of the thiol reactive coupling lipid MPB-PE in liposomes does not significantly affect their in vivo clearance behavior when compared to EPC/CHOL liposomes, suggest- ing that the binding of thiol-containing serum proteins does not affect the in vivo properties of liposomes.

### EXAMPLE 24

### Stability of Covalently Conjugated Liposomes In Vivo

The stability of covalently conjugated streptavidin-liposomes in vivo was demonstrated by the binding of biotin to liposome samples isolated from plasma 1 and 4 hours post injection (Table 6, below). A slight loss of biotin binding capacity of streptavidin-coupled liposomes was observed for samples isolated from plasma, which may have arisen from the absorption of serum components to the vesicles, the inactivation of streptavidin by proteolysis or the binding of endogenous biotin to the preparation.

**Table 6**

| Stability of Streptavidin-Liposome Conjugates In Vivo Streptavidin-Liposome - in ug Streptavidin/umol. Lipid Sample | | | |
|---|---|---|---|
| | Prior to Administration | After Administration | |
| | | 1 Hour | 4 hours |
| Aggregated (>530 nm) | 42.9 + 0.1 | 43.1 + 0.8 | 29.8 + 0.8 |
| Extruded (187 nm) | 41.1 + 2.8 | 35.4 + 0.2 | 32.9 + 0.3 |
| Extruded (139 nm) | 47.1 + 0.5 | 44.5 + 1.4 | 39.1 + 0.6 |

The amount of streptavidin attached to liposomes was determined by the binding of 14C biotin to lipid samples or pooled plasma samples from three mice, 1 and 4 hours post injection.

It will be understood by those skilled in the art that the foregoing description and examples are illustrative of practicing the present invention, but are in no way limiting. Variations of the detail presented herein may be made without departing from the spirit and scope of the present invention.

## Claims (Claims for the following Contracting State(s): AT, BE, CH, LI, DE, DK, FR, GB, IT, LU, NL, SE)

1. A reactive lipid comprising the reaction product of a nucleophilic liposome-forming lipid with a crosslinking agent containing at least one maleimide moiety, said reactive lipid being free of ring-opened crosslinking agent.

2. The reactive lipid according to claim 1, wherein said nucleophilic lipid is a phosphatidylethanolamine.

3. The reactive lipid according to claim 1 or 2, wherein the phosphatidylethanolamine is dipalmitoyl phosphatidylethanolamine, dimyristoyl p hosphatidylethanolamine, distearoyl phosphatidylethanolamine, dioleoyl phosphatidylethanolamine or egg phosphatidylethanolamine.

4. The reactive lipid according to anyone of claims 1 to 3, wherein said crosslinking agent contains an N-[4-(p-maleimidophenyl)-butyryl] MPB group.

5. The reactive lipid according to claim 4, wherein said crosslinking agent is N-succinimidyl 4-(p-maleimidophenyl)-butyrate (SMPB).

6. The reactive lipid according to claims 1or 5, comprising the reaction product of a phosphatidylethanolamine with N-succinimidyl 4-(p-maleimidophenyl) butyrate(SMPB) in the absence of a nucleophilic alcohol.

7. The reactive lipid according to claims 1 to 6, wherein said reaction product is MPB-DPPE, MPB-DMPE or MPB-EPE.

8. A liposome comprising the reactive lipid of any one of claims 1 to 7, wherein the reactive lipid comprises at least 0.05 mole percent of the lipid in the liposome.

9. The liposome according to claim 8, wherein the reactive lipid comprises from 0.05 mole percent to 2.5 mole percent of the lipid in the liposome.

10. The liposome according to anyone of claims 1 to 9 comprising a molecule conjugated to the reactive lipid, wherein the conjugated molecule is effective for therapeutic or diagnostic targeting of the liposome.

11. The conjugated liposome according to claim 10, wherein said conjugated molecule is a protein, an antibody or a cofactor.

12. The conjugated liposome according to claim 11, wherein said conjugated molecule is the protein streptavidin.

13. The conjugated liposome according to claim 11, wherein said antibody is a monoclonal antibody.

14. The conjugated liposome according to claim 11, wherein said cofactor is biotin.

15. The conjugated liposome according to claim 11, further comprising a protein noncovalently bound to said cofactor.

16. The conjugated liposome according to claim 15, wherein the cofactor is biotin and wherein streptavidin is noncovalently bound to said biotin.

17. The conjugated liposome according to anyone of claims 10 to 16, wherein said conjugated liposome contains a bioactive agent.

18. The conjugated liposome according to anyone of claims 10 to 17, wherein said conjugated liposome has an electrochemical potential across its outermost lipid bilayer.

19. The conjugated liposome according to anyone of claims 10 to 18, wherein the conjugated liposome is dehydrated.

20. The conjugated liposome according to anyone of claims 10 to 19, wherein said conjugated molecule is protein and the conjugated liposome is a stable, sized liposome exhibiting an absence of aggregation to other liposomes.

21. A pharmaceutical composition comprising the liposome of anyone of claims 17 to 20 and a pharmaceutically acceptable carrier or diluent.

22. The composition according to claim 21, wherein the bioactive agent is an antineoplastic agent.

23. The composition according to claim 22, wherein said antineoplastic agent is selected from the group consisting of daunorubicin, doxorubicin, vinblastine, vincristine, cisplatinum, cyclophosphamide and pharmaceutically acceptable salts and derivatives and mixtures, thereof.

24. A method of preparing a reactive lipid according to anyone of claims 2 to 23 which comprises:
a) reacting a nucleophilic liposome forming lipid with a crosslinking agent comprising a maleimide moiety in the presence of at least one non-nucleophilic solvent and in the absence of nucleophilic solvents so as to form a reaction solution for a period of time sufficient to complete conversion of the nucleophilic lipid to a reactive lipid ;
b) separating said reactive lipid from said solution by concentrating said solution in vacuo to produce a solid residue and triturating said solid residue with a non nucleophilic solvent and wherein said reactive lipid is free of lipids bound to ring-opened crosslinking agent.

25. The method according to claim 24 further comprising an extraction step following the reaction of step (a), wherein said reaction solution is diluted with said non-nucleophilic solvent and then washed at least one time to remove by-products.

26. The method according to claim 25, wherein said non-nucleophilic solvent is selected from the group consisting of chloroform, methylene chloride, DMF, DMA, DMSO, THF and 1,4-dioxane.

27. A stable, sized protein-liposome conjugate according to claim 20 having a lipid bilayer comprising :
(a) at least 90 mole percent of a liposome-forming lipid ;
(b) at least 0.1 mole percent of a functionalized lipid ; and
(c) a protein linked to said functionalized lipid in an amount equal to 10 to 100 protein molecules per liposome,
wherein said liposome exhibits an absence of aggregation to other liposomes.

28. The protein-liposome conjugate according to claim 27, wherein said functionalized lipid comprises between 0.25 mole percent and 1 mole percent of the lipid of said conjugate.

29. The protein-liposome conjugate of anyone of claims 27 or 28, wherein said protein is linked to said liposome in an amount equal to 55 to 80 protein molecules per liposome.

30. The protein-liposome conjugate according to anyone of claims 27 to 29, wherein said functionalized lipid is selected from the group consisting of MPB-phosphatidylethanolamine, PDP-phosphatidylethanolamine and biotin-phosphatidylethanolamine.

31. The protein-liposome conjugate according to anyone of claims 28 to 31, wherein said protein is linked by covalent bonds to said functionalized lipid.

32. The protein-liposome conjugate according to claim 31, wherein said covalent bonds are disulfide bonds.

33. The protein-liposome conjugate according to anyone of claims 27 to 32, wherein said protein is streptavidin, an antibody or an enzyme.

34. The protein-liposome conjugate according to claim 33, wherein the protein is streptavidin and wherein the streptavidin is additionally coupled to a biotinylated protein.

35. The protein-liposome conjugate according to claim 27, wherein said protein is linked by noncovalent bonds to said functionalized lipid.

36. The protein-liposome conjugate according to claim 35, wherein said functionalized lipid comprises biotin-phosphatidylethanolamine.

37. The protein-liposome conjugate according to anyone of claims 27 to 36, wherein the conjugate ranges in size from 75 nm to 200 nm.

38. The protein-liposome conjugate according to anyone of claims 27 to 37 comprising a bioactive agent.

39. The protein-liposome conjugate according to anyone of claims 27 to 38, wherein the conjugate is dehydrated.

40. The protein-liposome conjugate according to anyone of claims 27 to 39, wherein the conjugate has an electrochemical potential across its outermost lipid bilayer.

41. A method for producing a stable, sized protein-liposome conjugate according to anyone of claims 27 to 40 which comprises :
(a) preparing a liposome having a lipid bilayer comprising a functionalized lipid and a liposome-forming lipid ;
(b) conjugating a protein to the liposome so as to form a protein-liposome conjugate ;
(c) extruding said protein-liposome conjugate through a filter,
wherein the lipid bilayer comprises at least 0.1 mole of the functionalized lipid and at least 90 mole percent of the liposome-forming lipid, wherein the protein is conjugated to the liposome in an amount equal to 10 to 100 protein molecules per liposome and wherein the liposome exhibits an absence of aggregation to other liposomes.

42. The method according to claim 41, wherein the filter is a polycarbonate filter having a pore size of from 30 nm to 100 nm.

43. The method according to claim 42, wherein said extrusion step is performed under high pressure.

44. The stable, sized protein-liposome conjugate prepared according to the method of claim 41.

## Claims (Claims for the following Contracting State(s): ES)

1. A method for preparing a reactive lipid comprising reacting the product of a nucleophilic liposome-forming lipid with a crosslinking agent containing at least one maleimide moiety, said reactive lipid being free of ring-opened crosslinking agent.

2. The method according to claim 1, wherein said nucleophilic lipid is a phosphatidylethanolamine.

3. The mehod according to claim 1 or 2, wherein the phosphatidylethanolamine is dipalmitoyl phosphatidylethanolamine, dimyristoyl p hosphatidylethanolamine, distearoyl phosphatidylethanolamine, dioleoyl phosphatidylethanolamine or egg phosphatidylethanolamine.

4. The method according to anyone of claims 1 to 3, wherein said crosslinking agent contains an N-[4-(p-maleimidophenyl)-butyryl] MPB group.

5. The method according to claim 4, wherein said crosslinking agent is N-succinimidyl 4-(p-maleimidophenyl)-butyrate (SMPB).

6. The method according to claims 1 or 5, comprising reacting the product of a phosphatidylethanolamine with N-succinimidyl 4-(p-maleimidophenyl) butyrate(SMPB) in the absence of a nucleophilic alcohol.

7. The method according to claims 1 to 6, wherein said reaction product is MPB-DPPE, MPB-DMPE or MPB-EPE.

8. A method for preparing a liposome comprising the reactive lipid prepared according to a method of any one of claims 1 to 7, wherein the reactive lipid comprises at least 0.05 mole percent of the lipid in the liposome.

9. The method according to claim 8, wherein the reactive lipid comprises from 0.05 mole percent to 2.5 mole percent of the lipid in the liposome.

10. The method according to anyone of claims 1 to 9 comprising conjugating a molecule to the reactive lipid, wherein the conjugated molecule is effective for therapeutic or diagnostic targeting of the liposome.

11. The method according to claim 10, wherein said conjugated molecule is a protein, an antibody or a cofactor.

12. The method according to claim 11, wherein said conjugated molecule is the protein streptavidin.

13. The method according to claim 11, wherein said antibody is a monoclonal antibody.

14. The method according to claim 11, wherein said cofactor is biotin.

15. The method according to claim 11, further comprising a protein noncovalently bound to said cofactor.

16. The method according to claim 15, wherein the cofactor is biotin and wherein streptavidin is noncovalently bound to said biotin.

17. The method according to anyone of claims 10 to 16, wherein said conjugated liposome contains a bioactive agent.

18. The method according to anyone of claims 10 to 17, wherein said conjugated liposome has an electrochemical potential across its outermost lipid bilayer.

19. The method according to anyone of claims 10 to 18, wherein the conjugated liposome is dehydrated.

20. The method according to anyone of claims 10 to 19, wherein said conjugated molecule is protein and the conjugated liposome is a stable, sized liposome exhibiting an absence of aggregation to other liposomes.

21. A method for preparing pharmaceutical composition comprising admixing the liposome obtained by the method of anyone of claims 17 to 20 and a pharmaceutically acceptable carrier or diluent.

22. The method according to claim 21, wherein the bioactive agent is an antineoplastic agent.

23. The method according to claim 22, wherein said antineoplastic agent is selected from the group consisting of daunorubicin, doxorubicin, vinblastine, vincristine, cisplatinum, cyclophosphamide and pharmaceutically acceptable salts and derivatives and mixtures, thereof.

24. A method of preparing a reactive lipid according to anyone of claims 2 to 23 which comprises:
a) reacting a nucleophilic liposome forming lipid with a crosslinking agent comprising a maleimide moiety in the presence of at least one non-nucleophilic solvent and in the absence of nucleophilic solvents so as to form a reaction solution for a period of time sufficient to complete conversion of the nucleophilic lipid to a reactive lipid;
b) separating said reactive lipid from said solution by concentrating said solution in vacuo to produce a solid residue and triturating said solid residue with a non nucleophilic solvent and wherein said reactive lipid is free of lipids bound to ring-opened crosslinking agent.

25. The method according to claim 24 further comprising an extraction step following the reaction of step (a), wherein said reaction solution is diluted with said non-nucleophilic solvent and then washed at least one time to remove by-products.

26. The method according to claim 25, wherein said non-nucleophilic solvent is selected from the group consisting of chloroform, methylene chloride, DMF, DMA, DMSO, THF and 1,4-dioxane.

27. A method for preparing a stable, sized protein-liposome conjugate according to claim 20 wherein the lipid bilayer comprises :
(a) at least 90 mole percent of a liposome-forming lipid ;
(b) at least 0.1 mole percent of a functionalized lipid ; and
(c) a protein linked to said functionalized lipid in an amount equal to 10 to 100 protein molecules per liposome,
and wherein said liposome exhibits an absence of aggregation to other liposomes.

28. The method according to claim 27, wherein said functionalized lipid comprises between 0.25 mole percent and 1 mole percent of the lipid of said conjugate.

29. The method of anyone of claims 27 or 28, wherein said protein is linked to said liposome in an amount equal to 55 to 80 protein molecules per liposome.

30. The method according to anyone of claims 27 to 29, wherein said functionalized lipid is selected from the group consisting of MPB-phosphatidylethanolamine, PDP-phosphatidylethanolamine and biotin-phosphatidylethanolamine.

31. The method according to anyone of claims 28 to 31, wherein said protein is linked by covalent bonds to said functionalized lipid.

32. The method according to claim 31, wherein said covalent bonds are disulfide bonds.

33. The method according to anyone of claims 27 to 32, wherein said protein is streptavidin, an antibody or an enzyme.

34. The method according to claim 33, wherein the protein is streptavidin and wherein the streptavidin is additionally coupled to a biotinylated protein.

35. The method according to claim 27, wherein said protein is linked by noncovalent bonds to said functionalized lipid.

36. The method according to claim 35, wherein said functionalized lipid comprises biotin-phosphatidylethanolamine.

37. The method according to anyone of claims 27 to 36, wherein the conjugate ranges in size from 75 nm to 200 nm.

38. The method according to anyone of claims 27 to 37 comprising a bioactive agent.

39. The method according to anyone of claims 27 to 38, wherein the conjugate is dehydrated.

40. The method according to anyone of claims 27 to 39, wherein the conjugate has an electrochemical potential across its outermost lipid bilayer.

41. A method for producing a stable, sized protein-liposome conjugate according to anyone of claims 27 to 40 which comprises :
(a) preparing a liposome having a lipid bilayer comprising a functionalized lipid and a liposome-forming lipid ;
(b) conjugating a protein to the liposome so as to form a protein-liposome conjugate ;
(c) extruding said protein-liposome conjugate through a filter,
wherein the lipid bilayer comprises at least 0.1 mole of the functionalized lipid and at least 90 mole percent of the liposome-forming lipid, wherein the protein is conjugated to the liposome in an amount equal to 10 to 100 protein molecules per liposome and wherein the liposome exhibits an absence of aggregation to other liposomes.

42. The method according to claim 41, wherein the filter is a polycarbonate filter having a pore size of from 30 nm to 100 nm.

43. The method according to claim 42, wherein said extrusion step is performed under high pressure.

44. The stable, sized protein-liposome conjugate prepared according to the method of claim 41.

## Patentansprüche (Patentansprüche für folgende(n) Vertragsstaat(en): AT, BE, CH, LI, DE, DK, FR, GB, IT, LU, NL, SE)

1. Reaktives Lipid, das umfaßt das Reaktionsprodukt zwischen einem nucleophilen, Liposom-bildenden Lipid und einem Vernetzungsmittel, das mindestens einen Maleimid-Rest enthält, wobei das reaktive Lipid frei von einem Ring-geöffneten Vernetzungsmittel ist.

2. Reaktives Lipid nach Anspruch 1, worin das nucleophile Lipid ein Phosphatidylethanolamin ist.

3. Reaktives Lipid nach Anspruch 1 oder 2, worin das Phosphatidylethanolamin Dipalmitoyl-phosphatidylethanolamin, Dimyristoyl-phosphatidylethanolamin, Distearoyl-phosphatidylethanolamin, Dioleoyl-phosphatidylethanolamin oder Ei- Phosphatidylethanolamin ist.

4. Reaktives Lipid nach einem der Ansprüche 1 bis 3, worin das Vernetzungsmittel eine N-[4-(p-Maleimidophenyl)butyryl](MPB)-Gruppe enthält.

5. Reaktives Lipid nach Anspruch 4, worin das Vernetzungsmittel N-Succinimidyl-4-(p-maleimidophenyl)butyrat (SMPB) ist.

6. Reaktives Lipid nach den Ansprüchen 1 bis 5, das umfaßt das Reaktionsprodukt eines Phosphatidylethanolamins mit N-Succinimidyl-4-(p-maleimidophenyl)butyrat (SMPB) in Abwesenheit eines nucleophilen Alkohols.

7. Reaktives Lipid nach den Ansprüchen 1 bis 6, worin das Reaktionsprodukt MPB-DPPE, MPB-DMPE oder MPB-EPE ist.

8. Liposom, welches das reaktive Lipid nach einem der Ansprüche 1 bis 7 enthält, wobei das reaktive Lipid mindestens 0,05 Mol-% des Lipids in dem Liposom ausmacht.

9. Liposom nach Anspruch 1, worin das reaktive Lipid 0,05 bis 2,5 Mol-% des Lipids in dem Liposom ausmacht.

10. Liposom nach einem der Ansprüche 1 bis 9, das ein an das reaktive Lipid konjugiertes Molekül enthält, wobei das konjugierte Molekül wirksam ist für die therapeutische oder diagnostische Ansteuerung des Liposoms.

11. Konjugiertes Liposom nach Anspruch 10, worin das konjugierte Molekül ein Protein, ein Antikörper oder ein Cofaktor ist.

12. Konjugiertes Liposom nach Anspruch 11, worin das konjugierte Molekül das Protein Streptavidin ist.

13. Konjugiertes Liposom nach Anspruch 11, worin der Antikörper ein monoklonaler Antikörper ist.

14. Konjugiertes Liposom nach Anspruch 11, worin der Cofaktor Biotin ist.

15. Konjugiertes Liposom nach Anspruch 11, das außerdem ein Protein enthält, das an den Cofaktor nicht-kovalent gebunden ist.

16. Konjugiertes Liposom nach Anspruch 15, worin der Cofaktor Biotin ist und worin Streptavidin nicht-kovalent an das Biotin gebunden ist.

17. Konjugiertes Liposom nach einem der Ansprüche 10 bis 16, worin das konjugierte Liposom ein bioaktives Agens enthält.

18. Konjugiertes Liposom nach einem der Ansprüche 10 bis 17, worin das konjugierte Liposom ein elektrochemisches Potential gegenüber seiner äußersten Lipid-Doppelschicht aufweist.

19. Konjugiertes Liposom nach einem der Ansprüche 10 bis 18, worin das konjugierte Liposom dehydratisiert ist.

20. Konjugiertes Liposom nach einem der Ansprüche 10 bis 19, worin das konjugierte Molekül Protein ist und das konjugierte Liposom ein stabiles klassiertes Liposom ist, bei dem keine Aggregation an andere Liposome vorliegt.

21. Pharmazeutische Zusammensetzung, die das Liposom nach einem der Ansprüche 17 bis 20 und einen pharmazeutisch akzeptablen Träger oder Verdünnungsmittel enthält.

22. Zusammensetzung nach Anspruch 21, worin das bioaktive Agens ein antineoplastisches Agens ist.

23. Zusammensetzung nach Anspruch 22, worin das antineoplastische Agens ausgewählt wird aus der Gruppe, die besteht aus Daunorubicin, Doxorubicin, Vinblastin, Vincristin, Cisplatin, Cyclophosphamid und pharmazeutisch akzeptablen Salzen und Derivaten und Mischungen davon.

24. Verfahren zur Herstellung eines reaktiven Lipids nach einem der Ansprüche 2 bis 23, das umfaßt:
a) die Umsetzung eines nucleophilen Liposom-bildenden Lipids mit einem Vernetzungsmittel, das einen Maleimid-Rest enthält, in Gegenwart mindestens eines nicht-nucleophilen Lösungsmittels und in Abwesenheit von nucleophilen Lösungsmitteln zur Bildung einer Reaktionslösung für eine Zeitspanne, die ausreicht, um die Umwandlung des nucleophilen Lipids in ein reaktives Lipid zu vervollständigen;
b) die Abtrennung des reaktiven Lipids von der Lösung durch Konzentrieren der Lösung im Vakuum unter Bildung eines festen Rückstandes und das Verreiben des festen Rückstandes mit einem nicht-nucleophilen Lösungsmittel, wobei das reaktive Lipid frei von Lipiden ist, die an ein Ring-geöffnetes Vernetzungsmittel gebunden sind.

25. Verfahren nach Anspruch 24, das außerdem umfaßt eine Extraktionsstufe nach der Reaktion der Stufe (a), wobei die Reaktionslösung mit dem nicht-nucleophilen Lösungsmittel verdünnt und dann mindestens einmal gewaschen wird, um Nebenprodukte zu entfernen.

26. Verfahren nach Anspruch 25, worin das nicht-nucleophile Lösungsmittel ausgewählt wird aus der Gruppe, die besteht aus Chloroform, Methylenchlorid, DMF, DMA, DMSO, THF und 1,4-Dioxan.

27. Stabiles klassiertes Protein-Liposom-Konjugat nach Anspruch 20, das eine Lipid-Doppelschicht aufweist und umfaßt:
a) mindestens 90 Mol-% eines Liposom-bildenden Lipids;
b) mindestens 0,1 Mol-% eines funktionalisierten Lipids; und
c) ein Protein, das an das funktionalisierte Lipid gebunden ist, in einer Menge von 10 bis 100 Proteinmolekülen pro Liposom,
wobei das Liposom keine Aggregation an andere Liposome aufweist.

28. Protein-Liposom-Konjugat nach Anspruch 27, worin das funktionalisierte Lipid 0,25 bis 1 Mol-% des Lipids des genannten Konjugats ausmacht.

29. Protein-Liposom-Konjugat nach einem der Ansprüche 27 oder 28, worin das Protein an das Liposom gebunden ist in einer Menge von 55 bis 80 Proteinmolekülen pro Liposom.

30. Protein-Liposom-Konjugat nach einem der Ansprüche 27 bis 29, worin das funktionalierte Lipid ausgewählt wird aus der Gruppe, die besteht aus MPB- Phosphatidylethanolamin, PDP- Phosphatidylethanolamin und Biotin-Phosphatidylethanolamin.

31. Protein-Liposom-Konjugat nach einem der Ansprüche 28 bis 31, worin das Protein durch kovalente Bindungen an das funktionalisierte Lipid gebunden ist.

32. Protein-Liposom-Konjugat nach Anspruch 31, worin die kovalenten Bindungen die Sulfid-Bindungen sind.

33. Protein-Liposom-Konjugat nach einem der Ansprüche 27 bis 32, worin das Protein Streptavidin, ein Antikörper oder ein Enzym ist.

34. Protein-Liposom-Konjugat nach Anspruch 33, worin das Protein Streptavidin ist und das Streptavidin außerdem an ein biotinyliertes Protein gekuppelt ist.

35. Protein-Liposom-Konjugat nach Anspruch 27, worin das Protein durch nicht-kovalente Bindungen an das funktionalisierte Lipid gebunden ist.

36. Protein-Liposom-Konjugat nach Anspruch 35, worin das funktionalisierte Lipid Biotin-Phosphatidylethanolamin enthält.

37. Protein-Liposom-Konjugat nach einem der Ansprüche 27 bis 36, worin das Konjugat eine Größe in dem Bereich von 75 bis 200 nm hat.

38. Protein-Liposom-Konjugat nach einem der Ansprüche 27 bis 37, das ein bioaktives Agens enthält.

39. Protein-Liposom-Konjugat nach einem der Ansprüche 27 bis 38, worin das Konjugat dehydratisiert ist.

40. Protein-Liposom-Konjugat nach einem der Ansprüche 27 bis 39, worin das Konjugat ein elektrochemisches Potential gegenüber seiner äußersten Lipid-Doppelschicht aufweist.

41. Verfahren zur Herstellung eines stabilen, klassierten Protein-Liposom-Konjugats nach einem der Ansprüche 27 bis 40, das umfaßt
a) die Herstellung eines Liposoms mit einer Lipid-Doppelschicht, die ein funktionalisiertes Lipid und ein Liposom-bildendes Lipid umfaßt;
b) die Konjugation eines Proteins an das Liposom zur Bildung eines Protein-Liposom-Konjugats;
c) das Extrudieren des Protein-Liposom-Konjugats durch einen Filter,
wobei die Lipid-Doppelschicht mindestens 0,1 Mol-% des funktionalisierten Lipids und mindestens 90 Mol-% des Liposom-bildenden Lipids enthält, wobei das Protein an das Liposom konjugiert ist in einer Menge von 10 bis 100 Proteinmolekülen pro Liposom und wobei das Liposom keine Aggregation an andere Liposome aufweist.

42. Verfahren nach Anspruch 41, worin der Filter ein Polycarbonatfilter mit einer Porengröße von 30 bis 100 nm ist.

43. Verfahren nach Anspruch 42, worin die Extrusionsstufe unter hohem Druck durchgeführt wird.

44. Stabiles klassiertes Protein-Liposom-Konjugat, das nach dem Verfahren nach Anspruch 41 hergestellt worden ist.

## Patentansprüche (Patentansprüche für folgende(n) Vertragsstaat(en): ES)

1. Verfahren zur Herstellung eines reaktiven Lipids, das umfaßt die Umsetzung eines nucleophilen Liposom-bildenden Lipids mit einem Vernetzungsmittel, das mindestens einen Maleimid-Rest enthält, wobei das rekative Lipid frei von einem Ring-geöffneten Vernetzungsmittel ist.

2. Verfahren nach Anspruch 1, worin das nucleophile Lipid ein Phosphatidylethanolamin ist.

3. Verfahren nach Anspruch 1 oder 2, worin das Phosphatidylethanolamin Dipalmitoyl-phosphatidylethanolamin, Dimyristoyl-phosphatidylethanolamin, Distearoyl-phosphatidylethanolamin, Dioleoyl-phosphatidylethanolamin oder Ei- Phosphatidylethanolamin ist.

4. Verfahren nach einem der Ansprüche 1 bis 3, worin das Vernetzungsmittel eine N-[4-(p-Maleimidophenyl)butyryl](MPB)-Gruppe enthält.

5. Verfahren nach Anspruch 4, worin das Vernetzungsmittel N-Succinimidyl-4-(p-maleimidophenyl)butyrat (SMPB) ist.

6. Verfahren nach den Ansprüchen 1 bis 5, das umfaßt das Reaktionsprodukt eines Phosphatidylethanolamins mit N-Succinimidyl-4-(p-maleimidophenyl)butyrat (SMPB) in Abwesenheit eines nucleophilen Alkohols.

7. Verfahren nach den Ansprüchen 1 bis 6, worin das Reaktionsprodukt MPB-DPPE, MPB-DMPE oder MPB-EPE ist.

8. Verfahren zur Herstellung eines Liposoms nach einem der Ansprüche 1 bis 7, wobei das reaktive Lipid mindestens 0,05 Mol-% des Lipids in dem Liposom ausmacht.

9. Verfahren nach Anspruch 8, wobei das reaktive Lipid 0,05 bis 2,5 Mol-% des Lipids in dem Liposom ausmacht.

10. Verfahren nach einem der Ansprüche 1 bis 9, das umfaßt das Konjugieren eines Moleküls an das reaktive Lipid, wobei das konjugierte Molekül wirksam ist für die therapeutische oder diagnostische Ansteuerung des Liposoms.

11. Verfahren nach Anspruch 10, worin das konjugierte Molekül ein Protein, ein Antikörper oder ein Cofaktor ist.

12. Verfahren nach Anspruch 11, worin das konjugierte Molekül das Protein Streptavidin ist.

13. Verfahren nach Anspruch 11, worin der Antikörper ein monoklonaler Antikörper ist.

14. Verfahren nach Anspruch 11, worin der Cofaktor Biotin ist.

15. Verfahren nach Anspruch 11, das außerdem umfaßt ein Protein, das an den Cofaktor nicht-kovalent gebunden ist.

16. Verfahren nach Anspruch 15, worin der Cofaktor Biotin ist und worin Streptavidin nicht-kovalent an das Biotin gebunden ist.

17. Verfahren nach einem der Ansprüche 10 bis 16, worin das konjugierte Liposom ein bioaktives Agens enthält.

18. Verfahren nach einem der Ansprüche 10 bis 17, worin das konjugierte Liposom ein elektrochemisches Potential gegenüber seiner äußersten Lipid-Doppelschicht aufweist.

19. Verfahren nach einem der Ansprüche 10 bis 18, worin das konjugierte Liposom dehydratisiert ist.

20. Verfahren nach einem der Ansprüche 10 bis 19, worin das konjugierte Molekül Protein ist und das konjugierte Liposom ein stabiles klassiertes Liposom ist, bei dem keine Aggregation an andere Liposome vorliegt.

21. Verfahren zur Herstellung einer pharmazeutischen Zusammensetzung, das umfaßt das Mischen des nach dem Verfahren nach einem der Ansprüche 17 bis 20 erhaltenen Liposoms mit einem pharmazeutisch akzeptablen Träger oder Verdünnungsmittel.

22. Verfahren nach Anspruch 21, worin das bioaktive Agens ein antineoplastisches Agens ist.

23. Verfahren nach Anspruch 22, worin das antineoplastische Agens ausgewählt wird aus der Gruppe, die besteht aus Daunorubicin, Doxorubicin, Vinblastin, Vincristin, Cisplatin, Cyclophosphamid und pharmazeutisch akzeptablen Salzen und Derivaten und Mischungen davon.

24. Verfahren zur Herstellung eines reaktiven Lipids nach einem der Ansprüche 2 bis 23, das umfaßt:
a) die Umsetzung eines nucleophilen Liposom-bildenden Lipids mit einem Vernetzungsmittel, das einen Maleimid-Rest enthält, in Gegenwart mindestens eines nicht-nucleophilen Lösungsmittels und in Abwesenheit von nucleophilen Lösungsmitteln zur Bildung einer Reaktionslösung für eine Zeitspanne, die ausreicht, um die Umwandlung des nucleophilen Lipids in ein reaktives Lipid zu vervollständigen;
b) die Abtrennung des reaktiven Lipids von der Lösung durch Konzentrieren der Lösung im Vakuum unter Bildung eines festen Rückstandes und das Verreiben des festen Rückstandes mit einem nicht-nucleophilen Lösungsmittel, wobei das reaktive Lipid frei von Lipiden ist, die an ein Ring-geöffnetes Vernetzungsmittel gebunden sind.

25. Verfahren nach Anspruch 24, das außerdem umfaßt eine Extraktionsstufe nach der Reaktion der Stufe (a), wobei die Reaktionslösung mit dem nicht-nucleophilen Lösungsmittel verdünnt und dann mindestens einmal gewaschen wird, um Nebenprodukte zu entfernen.

26. Verfahren nach Anspruch 25, worin das nicht-nucleophile Lösungsmittel ausgewählt wird aus der Gruppe, die besteht aus Chloroform, Methylenchlorid, DMF, DMA, DMSO, THF und 1,4-Dioxan.

27. Verfahren zur Herstellung eines stabilen, klassiertes Protein-Liposom-Konjugats nach Anspruch 20, wobei die Lipid-Doppelschicht enthält:
a) mindestens 90 Mol-% eines Liposom-bildenden Lipids;
b) mindestens 0,1 Mol-% eines funktionalisierten Lipids; und
c) ein Protein, das an das funktionalisierte Lipid gebunden ist, in einer Menge von 10 bis 100 Proteinmolekülen pro Liposom,
wobei das Liposom keine Aggregation an andere Liposome aufweist.

28. Verfahren nach Anspruch 27, worin das funktionalisierte Lipid 0,25 bis 1 Mol-% des Lipids des genannten Konjugats ausmacht.

29. Verfahren nach einem der Ansprüche 27 oder 28, worin das Protein an das Liposom gebunden ist in einer Menge von 55 bis 80 Proteinmolekülen pro Liposom.

30. Verfahren nach einem der Ansprüche 27 bis 29, worin das funktionalierte Lipid ausgewählt wird aus der Gruppe, die besteht aus MPB- Phosphatidylethanolamin, PDP- Phosphatidylethanolamin und Biotin- Phosphatidylethanolamin.

31. Verfahren nach einem der Ansprüche 28 bis 31, worin das Protein durch kovalente Bindungen an das funktionalisierte Lipid gebunden ist.

32. Verfahren nach Anspruch 31, worin die kovalenten Bindungen die Sulfid-Bindungen sind.

33. Verfahren nach einem der Ansprüche 27 bis 32, worin das Protein Streptavidin, ein Antikörper oder ein Enzym ist.

34. Verfahren nach Anspruch 33, worin das Protein Streptavidin ist und das Streptavidin außerdem an ein biotinyliertes Protein gekuppelt ist.

35. Verfahren nach Anspruch 27, worin das Protein durch nicht-kovalente Bindungen an das funktionalisierte Lipid gebunden ist.

36. Verfahren nach Anspruch 35, worin das funktionalisierte Lipid Biotin-Phosphatidylethanolamin umfaßt.

37. Verfahren nach einem der Ansprüche 27 bis 36, worin das Konjugat eine Größe in dem Bereich von 75 bis 200 nm hat.

38. Verfahren nach einem der Ansprüche 27 bis 37, das ein bioaktives Agens umfaßt.

39. Verfahren nach einem der Ansprüche 27 bis 38, worin das Konjugat dehydratisiert ist.

40. Verfahren nach einem der Ansprüche 27 bis 39, worin das Konjugat ein elektrochemisches Potential gegenüber seiner äußersten Lipid-Doppelschicht aufweist.

41. Verfahren zur Herstellung eines stabilen, klassierten Protein-Liposom-Konjugats nach einem der Ansprüche 27 bis 40, das umfaßt
a) die Herstellung eines Liposoms mit einer Lipid-Doppelschicht, die ein funktionalisiertes Lipid und ein Liposom-bildendes Lipid umfaßt;
b) die Konjugation eines Proteins an das Liposom zur Bildung eines Protein-Liposom-Konjugats;
c) das Extrudieren des Protein-Liposom-Konjugats durch einen Filter,
wobei die Lipid-Doppelschicht mindestens 0,1 Mol-% des funktionalisierten Lipids und mindestens 90 Mol-% des Liposom-bildenden Lipids enthält, wobei das Protein an das Liposom konjugiert ist in einer Menge von 10 bis 100 Proteinmolekülen pro Liposom und wobei das Liposom keine Aggregation an andere Liposome aufweist.

42. Verfahren nach Anspruch 41, worin der Filter ein Polycarbonatfilter mit einer Porengröße von 30 bis 100 nm ist.

43. Verfahren nach Anspruch 42, worin die Extrusionsstufe unter hohem Druck durchgeführt wird.

44. Stabiles klassiertes Protein-Liposom-Konjugat, das nach dem Verfahren nach Anspruch 41 hergestellt worden ist.

## Revendications (Revendications pour l'(les) Etat(s) contractant(s) suivant(s): AT, BE, CH, LI, DE, DK, FR, GB, IT, LU, NL, SE)

1. Un lipide réactif comportant le produit de la réaction entre un lipide nucléophile formant des liposomes et un agent réticulant comportant au moins une partie maléimide, ledit lipide réactif étant exempt d'agents réticulant à ouverture de cycle.

2. Le lipide réactif selon la revendication 1, dans lequel ledit lipide nucléophile est une phosphatidyléthanolamine.

3. Le lipide réactif selon la revendication 2, dans lequel la phosphatidyléthanolamine est une dipalmitoyl-phosphatidyléthanolamine, une dimyristoyl-phospnatidyléthanolamine, une distéaroyl-phosphatidyléthanolarnine, une dioléoyl-phosphatidyléthanolamine ou une phosphatidyléthanolamine de l'oeuf.

4. Le lipide réactif selon l'une quelconque des revendications 1 à 3, dans lequel ledit agent de réticulation renferme un groupe N-[4-(p-maléimidophényl)-butyryl] (MPB).

5. Le lipide réactif selon la revendication, 4, dans lequel ledit agent réticulant le 4-(p-maléimidophényl)-butyrate de N-succinimidyle (SMPB).

6. Le lipide réactif selon les revendications 1 ou 5, comportant le produit de la réaction entre une phosphatidyléthanolamine et du 4-(p-maléimidophényl)-butyrate de N-succinimidyle (SMPB) en l'absence d'un alcool nucléophile.

7. Le lipide réactif selon les revendications 1 à 6, dans lequel ledit produit de réaction est la N-[p-maléimidophényl)-butyryl] dipalmitoyl-phosphatidyléthanolamine (MPB-DPPE), la N-[4-(p-maléimidophényl)-butyryl]-dimyristoyl - phosphatidyléthanolamine (DMPE) et la N-[4-(p-maléimidophényl)-butyryl] phosphatidyléthanolamine (EPE).

8. Un liposome comportant le lipide réactif de l'une quelconque des revendications 1 à 7, dans lequel le lipide réactif comporte au moins 0,05 pour-cent molaire du lipide dans le liposome.

9. Le liposome selon la revendication 8, dans lequel le lipide réactif comporte 0,05 pour-cent molaire à 2,5 pour-cent molaire du lipide dans le liposome.

10. Le liposome selon l'une quelconque des revendications 1 à 9, comportant une molécule conjuguée au lipide réactif, dans lequel la molécule conjuguée est efficace pour le ciblage thérapeutique ou diagnostic du liposome.

11. Le liposome conjugué selon la revendication 10, dans lequel la molécule conjuguée est une protéine, un anticorps ou un co-facteur.

12. Le liposome conjugué selon la revendication 11, dans lequel ladite molécule conjuguée est la protéine streptavidine.

13. Le liposome conjugué selon la revendication 11, dans lequel l'anticorps est un anticorps monoclonal.

14. Le liposome conjugué selon la revendication 11, dans lequel ledit co-facteur est la biotine.

15. Le liposome conjugué selon la revendication 11, comportant en outre une protéine non-covalente liée audit co-facteur.

16. Le liposome conjugué selon la revendication 15, dans lequel le co-facteur est la biotine et dans lequel la streptavidine est liée, de façon non-covalente, à ladite biotine.

17. Le liposome conjugué selon l'une quelconque des revendications 10 à 16, dans lequel ledit liposome conjugué renferme un agent bioactif.

18. Le liposome conjugué selon l'une quelconque des revendications 10 à 17, dans lequel ledit liposome conjugué présente un potentiel électrochimique à travers sa bicouche lipidique extérieure.

19. Le liposome conjugué selon l'une quelconque des revendications 10 à 18, dans lequel le liposome conjugué est déshydraté.

20. Le liposome conjugué selon l'une quelconque des revendications 10 à 19, dans lequel ladite molécule conjuguée est une protéine et le liposome conjugué est un liposome stable, dimensionné, qui présente une absence d'agrégation aux autres liposomes.

21. Une composition pharmaceutique comportant le liposome de l'une quelconque des revendications 17 à 20 et un support ou un diluant pharmaceutiquement acceptable.

22. La composition selon la revendication 21, dans laquelle l'agent bioactif est un agent anti-néoplastique.

23. La composition selon la revendication 22, dans laquelle ledit agent anti-néoplastique est choisi dans le groupe constitué de la daunorubicine, la doxorubicine, la vinblastine, la vincristine, le cisplatine, le cyclophosphamide et des sels et des dérivés pharmaceutiquement acceptables, ainsi que des mélanges de ceux-ci.

24. Un procédé pour préparer un lipide réactif selon l'une quelconque des revendications 2 à 23 , qui comporte les étapes qui consistent :
a) à faire réagir un lipide nucléophile formant liposome avec un agent réticulant comportant une partie maléimide en présence d' au moins un solvant non-nucléophile et en l'absence de solvant nucléophile de façon à former une solution de réaction pendant une période de temps suffisante pour terminer la conversion dit lipide nucléophile en lipide réactif;
b) à séparer ledit lipide réactif de ladite solution en concentrant ladite solution sous vide afin d'obtenir un résidu solide et en triturant ledit résidu solide avec un solvant non-nucléophile et dans lequel ledit lipide réactif est exempt de lipides liés à l'agent réticulant à ouverture de cycle.

25. Le procédé selon la revendication 24, comportant en outre une étape d'extraction à la suite de la réaction de l'étape (a), dans lequel ladite solution de réaction est diluée avec ledit solvant non-nucléopllile et est ensuite lavé au moins une fois pour éliminer les produits secondaires.

26. Le procédé selon la revendication 25, dans lequel ledit solvant nucléophile est choisi dans le groupe constitué du chloroforme, du chlorure de méthylène, du diméthylformamide, du diméthylacétamide, du diméthylsulfoxyde, du tétrahydrofluorane et du 1,4-dioxane.

27. Un conjugué dimensionné protéine-liposome selon la revendication 20, possédant une bicouche lipidique comportant:
(a) au moins 90 pour-cent molaire d'un lipide formant un liposome;
(b) au moins 0,1 pour-cent molaire d'un lipide fonctionnel, et
(c) une protéine liée audit lipide fonctionnel en une quantité égale de 10 à 100 molécules protéiniques par liposome,
dans lequel ledit liposome présente une absence d'agrégation aux autres liposomes.

28. Le conjugué protéine-liposome selon la revendication 27, dans lequel ledit lipide fonctionnel comporte entre 0,25 pour-cent molaire et 1 pour-cent molaire de lipide dudit conjugué.

29. Le conjugué protéine-liposome selon les renvendications 27 ou 28, dans lequel ladite protéine est lié audit liposome en une quantité égale à de 55 à 80 molécules de protéine par liposome.

30. Le conjugué protéine-liposome selon l'une quelconque des revendications 27 à 29, dans lequel ledit lipide fonctionnel est choisi dans le groupe constitué de la MPB-phosphatidyléthanolamine, de la PDP-phosphatidyléthanolamine et de la biotine-phosphatidyléthanolamine.

31. Le conjugué protéine-liposome selon l'une quelconque des revendications 28 à 31, dans lequel ladite protéine est liée par des liaisons covalentes audit lipide fonctionnel.

32. Le conjugué protéine-liposome selon la revendication 31, dans lequel les liaisons covalentes sont des liaisons disulfure.

33. Le conjugué protéine-liposome selon l'une quelconque des revendications 27 à 32, dans lequel ladite protéine est la streptavidine, un anticorps ou un enzyme.

34. Le conjugué protéine-liposome selon la revendication 33, dans lequel la protéine est la streptavidine et dans lequel la streptavidine est couplée, en outre, à la protéine biotinylée.

35. Le conjugué protéine-liposome selon la revendication 27, dans lequel ladite protéine est liée par liaison non-covalente audit lipide fonctionnel.

36. Le conjugué protéine-liposome selon la revendication 35, dans lequel ledit lipide fonctionnel comporte de la biotine-phosphatidyléthanol amine.

37. Le conjugué protéine-liposome selon l'une quelconque des revendications 27 à 36, dans lequel le conjugué se situe dans la gamme dimensionnelle de 75 nm à 200 nm.

38. Le conjugué protéine-liposome selon l'une quelconque des revendications 27 à 37 comportant un agent bioactif.

39. Le conjugué protéine-liposome selon l'une quelconque des revendications 27 à 38, dans lequel le conjugué est déshydraté.

40. Le conjugué protéine-liposome selon l'une quelconque des revendications 27 à 39 dans lequel le conjugué présente un potentiel électrochimique à travers sa bicouche lipidique extérieure.

41. Un procédé pour préparer un conjugué protéine-liposome stable et dimensionné, selon l'une quelconque des revendications 27 à 40, et qui comprend les étapes qui consistent :
(a) à préparer un liposome présentant une bicouche lipidique comportant un lipide fonctionnel et un lipide formant un liposome;
(b) à conjuguer une protéine au liposome de façon à former un conjugué protéine-liposome;
(c) à extruder ledit conjugué protéine-liposome à travers un filtre, dans lequel la bicouche lipidique comporte au mois 0,1 mole de
lipide fonctionnel et au moins 90 pour-cent molaire du lipide formant un liposome, la protéine étant conjuguée au liposome en une quantité égale à de 10 à 100 molécules de protéine par liposome, tandis que le liposome présente une absence d'agrégation aux autres liposomes .

42. Le procédé selon la revendication 41, dans lequel le filtre est un filtre à base de polycarbonate, qui présente une dimension des pores de 30 nm à 100 nm.

43. Le procédé selon la revendication 42, dans lequel ladite étape d'extrusion est réalisée sous pression élevée.

44. Le conjugué protéine-liposome, dimensionné et stable, préparé selon le procédé de la revendication 41.

## Revendications (Revendications pour l'(les) Etat(s) contractant(s) suivant(s): ES)

1. Un procédé pour préparer un lipide réactif comportant le produit de la réaction entre un lipide nucléophile formant des liposomes et un agent réticulant comportant au moins une partie maléimide, ledit lipide réactif étant exempt d'agents réticulant à ouverture de cycle.

2. Le procédé selon la revendication 1, dans lequel ledit lipide nucléophile est une phosphatidyléthanolamine.

3. Le procédé selon la revendication 1 ou 2, dans lequel la phosphatidyléthanolamine est une dipalmitoyl-phosphatidyléthanolamine, une dimyristoyl-phosphatidyléthanolamine, une distéaroyl-phosphatidyléthanolamine une dioléoyl-phosphatidyléthanolamine ou une phosphatidyléthanolamine de l'oeuf.

4. Le procédé selon l'une quelconque des revendications 1 à 3, dans lequel ledit agent de réticulation renferme un groupe N-[4-(p-maléimidophényl)-butyryl] (MPB).

5. Le procédé selon la revendication 4, dans lequel ledit agent réticulant le 4-(p-maléimidophényl)-butyrate de N-succinimidyle (SMPB).

6. Le procédé selon les revendications 1 ou 5, comportant le produit de la réaction entre une phosphatidyléthanolamine et du 4-(p-maléimidophényl)-butyrate de N-succinimidyle (SMPB) en l'absence d'un alcool nucléophile.

7. Le procédé selon les revendications 1 à 6, dans lequel ledit produit de réaction est la N-[p-maléimidophényl)-butyryl] dipalmitoyl-phosphatidyléthanolamine (MPB-DPPE), la N-[4-(p-maléimidophényl)-butyryl]-dimyristoyl - phosphatidyléthanolamine (DMPE) et la N-[4-(p-maléimidophényl)-butyryl] phosphatidyléthanolamine (EPE).

8. Un procédé pour préparer un liposome comportant le lipide réactif préparé selon un procédé de l'une quelconque des revendications 1 à 7, dans lequel le lipide réactif comporte au moins 0,05 pour-cent molaire du lipide dans le liposome.

9. Le procédé selon la revendication 8, dans lequel le lipide réactif comporte 0,05 pour-cent molaire à 2,5 pour-cent molaire du lipide dans le liposome.

10. Le procédé selon l'une quelconque des revendications 1 à 9, comportant une molécule conjuguée au lipide réactif, dans lequel la molécule conjuguée est efficace pour le ciblage thérapeutique ou diagnostic du liposome.

11. Le procédé selon la revendication 10, dans lequel la molécule conjuguée est une protéine, un anticorps ou un co-facteur.

12. Le procédé selon la revendication 11, dans lequel ladite molécule conjuguée est la protéine streptavidine.

13. Le procédé selon la revendication 11, dans lequel ledit anticorps est un anticorps monoclonal.

14. Le procédé selon la revendication 11, dans lequel ledit co-facteur est la biotine.

15. Le procédé selon la revendication 11, comportant en outre une protéine liée de façon non-covalente audit co-facteur.

16. Le procédé selon la revendication 15, dans lequel le co-facteur est la biotine et dans lequel la streptavidine est liée, de façon non-covalente, à ladite biotine.

17. Le procédé selon l'une quelconque des revendications 10 ou 16, dans lequel ledit liposome conjugué renferme un agent bioactif.

18. Le procédé selon l'une quelconque des revendications 10 à 17, dans lequel ledit liposome conjuguée présente un potentiel électrochimique à travers sa bicouche lipidique extérieure.

19. Le procédé selon l'une quelconque des revendications 10 à 18, dans lequel le liposome conjugué est déshydraté.

20. Le procédé selon l'une quelconque des revendications 10 à 19, dans lequel ladite molécule conjuguée est une protéine et le liposome conjugué est un liposome stable, dimensionné, qui présente une absence d'agrégation aux autres liposomes.

21. Un procédé pour préparer une composition pharmaceutique, consistant à mélanger le liposome obtenu par l'une quelconque des revendications 17 à 20 et un support ou bien un diluant pharmaceutiquement acceptable.

22. Le procédé selon la revendication 21, dans lequel l'agent bioactif est un agent anti-néoplastique.

23. Le procédé selon la revendication 22, dans laquelle ledit agent anti-néoplastique est choisi dans le groupe constitué de la daunorubicine, la doxorubicine, la vinblastine, la vincristine, le cisplatine, le cyclophosphamide et des sels et des dérivés pharmaceutiquement acceptables, ainsi que des mélanges de ceux-ci.

24. Un procédé pour préparer un lipide réactif selon l'une quelconque des revendications 2 à 23, qui consiste:
a) à faire réagir un lipide nucléophile formant liposome avec un agent réticulant comportant une partie maléimide en présence d'au moins un solvant non-nucléophile et en l'absence de solvant nucléophile de façon à former une solution de réaction pendant une période de temps suffisante pour terminer la conversion du lipide nucléophile en lipide réactif;
b) à séparer ledit lipide réactif de ladite solution en concentrant ladite solution sous vide afin d'obtenir un résidu solide et en triturant ledit résidu solide avec un solvant non-nucléophile et dans lequel ledit lipide réactif est exempt de lipides liés à l'agent réticulant à ouverture de cycle.

25. Le procédé selon la revendication 24, comportant en outre une étape d'extraction à la suite de la réaction de l'étape (a), dans lequel ladite solution de réaction est dilué avec ledit solvant non-nucléophile et est ensuite lavé au moins une fois pour éliminer les produits secondaires.

26. Le procédé selon la revendication 25, dans lequel ledit solvant nucléophile est choisi dans le groupe constitué du chloroforme, du chlorure de méthylène, du diméthylformamide, du diméthylacétamide, du diméthylsulfoxyde, du tétrahydrofluorane et du 1,4-dioxane.

27. Un procédé préparé, conjugué dimensionné protéineliposome selon la revendication 20, possédant une bicouche lipidique comportant:
(a) au moins 90 pour-cent molaire d'un lipide formant un liposome;
(b) au moins 0,1 pour-cent molaire d'un lipide fonctionnel, et
(c) une protéine liée audit lipide fonctionnel en une quantité égale de 10 à 100 molécules protéiniques par liposome,
dans lequel ledit liposome présente une absence d'agrégation aux autres liposomes.

28. Le procédé selon la revendication 27, dans lequel ledit lipide fonctionnel comporte entre 0,25 pour-cent molaire et 1 pour-cent molaire de lipide dudit conjugue.

29. Le procédé de l'une quelconque des renvendications 27 ou 28, dans lequel ladite protéine est lié audit liposome en une quantité égale à de 55 à 80 molécules de protéine par liposome.

30. Le procédé selon l'une quelconque des revendications 27 à 29, dans lequel ledit lipide fonctionnel est choisi dans le groupe constitué de la MPB-phospliatidyléthanolamine, de la PDP-phosphatidyléthanolamine et de la biotine-phosphàtidyléthanolamine.

31. Le procédé selon l'une quelconque des revendications 28 à 31, dans lequel ladite protéine est liée par des liaisons covalentes audit lipide fonctionnel.

32. Le procédé selon la revendication 31, dans lequel les liaisons covalentes sont des liaisons disulfure.

33. Le procédé selon l'une quelconque des revendications 27 à 32, dans lequel ladite protéine est la streptavidine, un anticorps ou un enzyme.

34. Le procédé selon la revendication 33, dans lequel la protéine est la streptavidine et dans lequel la streptavidine est couplée, en outre, à la protéine biotinylée.

35. Le procédé selon la revendication 27, dans lequel ladite protéine est liée par des liaisons non-covalentes audit lipide fonctionnel.

36. Le procédé selon la revendication 35, dans lequel ledit lipide fonctionnel comporte de la biotine-phosphatidyléthanolamine.

37. Le procédé selon l'une quelconque des revendications 27 à 36, dans lequel le conjugué se situe dans la plage dimensionnelle de 75 nm à 200 nm.

38. Le procédé selon l'une quelconque des revendications 27 à 37, comportant un agent bioactif.

39. Le procédé selon l'une quelconque des revendications 27 à 38, dans lequel le conjugué est déshydraté.

40. Le procédé selon l'une quelconque des revendications 27 à 39, dans lequel le conjugué présente un potentiel électrochimique à travers sa bicouche lipidique extérieure.

41. Un procédé pour préparer un conjugué protéine-liposome stable et dimensionné, selon l'une quelconque des revendications 27 à 40, et qui consiste:
(a) à préparer un liposome présentant une bicouche lipidique comportant un lipide fonctionnel et un lipide formant un liposome;
(b) à conjuguer une protéine au liposome de façon à former un conjugué protéine-liposome;
(c) à extruder ledit conjugué protéine-liposome à travers un filtre,
dans lequel la bicouche lipidique comporte au mois 0,1 mole de lipide fonctionnel et au moins 90 pour-cent molaire du lipide formant un liposome, la protéine étant conjuguée au liposome en une quantité égale à de 10 à 100 molécules de protéine par liposome, tandis que le liposome présente une absence d'agrégation aux autres liposomes.

42. Le procédé selon la revendication 41, dans lequel le filtre est un filtre à base de polycarbonate, qui présente une dimension des pores de 30 nm à 100 nm.

43. Le procédé selon la revendication 42, dans lequel ladite étape d'extrusion est réalisée sous pression élevée.

44. Le conjugué protéine-liposome, dimensionné et stable, préparé selon le procédé de la revendication 41.
